# EUROPEAN PATENT SPECIFICATION

(11) **EP 3 515 503 B1**
(45) Date of publication and mention of the grant of the patent: **29.10.2025**
(21) Application number: 17854002.7
(22) Date of filing: 22.09.2017
(51) Int. Cl.: A61K 39/395, C07K 16/30, C07K 16/28, C07K 16/46, A61K 40/33, A61K 40/42, C07K 14/725, C07K 14/705, C12N 5/0783, G01N 33/574, A61K 40/11, A61K 40/15, A61K 40/31, A61P 35/02

(54) **ENGINEERED LYMPHOCYTES**
MANIPULIERTE LYMPHOZYTEN
LYMPHOCYTES MODIFIÉS

(30) Priority: 23.09.2016 US 201662398859 P
(43) Date of publication of application: 31.07.2019
(73) Proprietor: The Regents of The University of Michigan, Ann Arbor, Michigan 48109 (US); Baylor College of Medicine, Houston, TX 77030 (US)
(72) Inventor: BONIFANT, Challice, Ann Arbor Michigan 48109 (US); GOTTSCHALK, Stephen M.G., Houston Texas 77030 (US)
(74) Representative: Glawe, Delfs, Moll
(86) International application number: PCT/US2017/052989
(87) International publication number: WO 2018/057915

(56) References cited:
- WO-A1-2014/051433
- WO-A1-2016/014535
- WO-A1-2016/120218
- WO-A2-2015/142675
- US-A1- 2017 281 766
- CHALLICE L BONIFANT ET AL: "CD123-Engager T Cells as a Novel Immunotherapeutic for Acute Myeloid Leukemia", MOLECULAR THERAPY : THE JOURNAL OF THE AMERICAN SOCIETY OF GENE THERAPY, vol. 24, no. 9, 1 September 2016 (2016-09-01), US, pages 1615 - 1626, XP055692154, ISSN: 1525-0016, DOI: 10.1038/mt.2016.116
- STEIN, C ET AL.: "Novel conjugates of single-chain Fv antibody fragments specific for stem cell antigen CD 123 mediate potent death of acute myeloid leukaemia cells", BRITISH JOURNAL OF HAEMATOLOGY, vol. 148, no. 6, March 2010 (2010-03-01), pages 879 - 889, XP002618076, [retrieved on 20100108], DOI: doi:10.1111/J.1365-2141.2009.08033.X
- LU , H ET AL.: "Targeting Human C-Type Lectin-Like Molecule-1 (CLL1) with a Bispecific Antibody for Acute Myeloid Leukemia Immunotherapy", ANGEWANDTE CHEMIE INTERNATIONAL EDITION ENGLAND, vol. 53, no. 37, 8 September 2014 (2014-09-08), pages 9841 - 9845, XP002752433, [retrieved on 20140723], DOI: doi:10.1002/anie.201405353
- LEONG, SR ET AL.: "An anti- CD 3/anti-CLL-1 bispecific antibody for the treatment of acute myeloid leukemia", BLOOD, vol. 129, no. 5, 2 February 2017 (2017-02-02), pages 609 - 618, XP055360863, [retrieved on 20161201], DOI: doi:10.1182/blood-2016-08-735365
- TASHIRO, H ET AL.: "Treatment of Acute Myeloid Leukemia with T Cells Expressing Chimeric Antigen Receptors Directed to C-type Lectin-like Molecule 1", MOLECULAR THERAPY, vol. 25, no. 9, 6 September 2017 (2017-09-06), pages 2202 - 2213, XP055579188, [retrieved on 20170701], DOI: doi:10.1016/j.ymthe.2017.05.024

## Description

### CROSS-REFERENCE TO RELATED APPLICATIONS

The present invention claims priority to U.S. Provisional Patent Application 62/398,859, filed September 23, 2016.

### FIELD

Provided here are compositions comprising engineered lymphocytes that secrete bispecific engager molecules, thereby activating T cells in the local environment to kill target cells. Engineered lymphocytes may selectively target CLL-1 positive leukemic cells, both directly and through activation of a subject's own T cells, while sparing CLL-1 negative cells, such as myeloid progenitor cells. Engineered lymphocytes may selectively target CD123 and CLL-1 positive leukemic cells, both directly and through activation of native T cells.

### BACKGROND

AML, with a cure rate of less than 30% for high risk and relapsed disease, is a cancer in need of new therapeutic strategies to impact patient survival. Despite the dismal prognosis, the mortality rate due to acute myeloid leukemia (AML) has remained relatively unchanged for the last two decades. The standard chemotherapy regimen has not been altered in nearly 40 years, in part because of the difficulty in specifically targeting leukemic myeloblasts while sparing normal tissue.

Bispecific engager to treat AML are known in the prior art, see e.g. Challice L Bonifant et al: "CD123-Engager T Cells as a Novel Immunotherapeutic for Acute Myeloid Leukemia", Molecular therapy: the journal of the American Society of Gene Therapy, vol.24, no.9, 1 September 2016, pages 1615-1626. Said document discloses T cells secreting a CD123 bispecific engager to treat AML. The claimed subject-matter differs from said prior art in that the engager expressed by the T cells binds CLL-1, more specifically, the bispecific engager molecule comprises a single chain variable fragment (scFv) activation domain that binds CD3 and an scFv antigen-recognition domain that binds CLL-1. Said feature also distinguishes the claimed invention over further prior art, e.g. WO 2014/051433 A1 disclosing the induction of T cell mediated killing of AML tumor cells in vitro with a CLEC12 (CLL-1)-CD3 bispecific antibody, LU, H et al.: 'Targeting Human C-Type Lectin-Like Molecule-1 (CLL1) with a Bispecific Antibody for Acute Myeloid Leukemia Immunotherapy", Angewandte Chemie International Edition England, vol. 53, no. 37, 8 September 2014, pages 9841 -9845, disclosing a bispecific antibody CLL-1-CD3 which can recruit T cells and eradicate AML in xenograft models, STEIN, C et al: "Novel conjugates of single-chain Fv antibody fragments specific for stem cell antigen CD 123 mediate potent death of acute myeloid leukaemia cells", British Journal of Haematology, vol. 148, no. 6, March 2010, pages 879-889, which discloses a CD123 scFv to target AML and WO 2016/120218 A1, WO 2015/142675 A2, TASHIRO, H et al.: "Treatment of Acute Myeloid Leukemia with T Cells Expressing Chimeric Antigen Receptors Directed to C-type Lectin-like Molecule 1", Molecular Therapy, vol. 25, no. 9, 6 September 2017, pages 2202-2213, and US 2017/281766 A1 which all relate to CLL-1 CARs.

### SUMMARY

The invention is set out in the appended claims. The embodiments of the description which do not fall within the scope of said claims are provided for illustrative purposes only and do not form part of the present invention. Provided here are compositions comprising engineered lymphocytes that secrete bispecific engager molecules, thereby activating T cells in the local environment to kill target cells. As described herein, engineered lymphocytes selectively target CLL-1 positive leukemic cells, both directly and through activation of a subject's own T cells, while sparing CLL-1 negative cells, such as myeloid progenitor cells.

The invention claims engineered lymphocytes as defined in the claims, including engineered lymphocytes for use in a method of treating acute myeloid leukemia (AML) in a subject, wherein said engineered lymphocytes express a polypeptide construct comprising an antigen-recognition domain and an activation domain, wherein the antigen-recognition domain binds C-type lectin-like molecule-1 (CLL-1) displayed on a malignant myeloblast cell, and wherein the activation domain triggers an immune response against the malignant myeloblast cell by the engineered lymphocytes and/or native T cells upon binding of the antigen-recognition domain to CLL-1, and wherein the polypeptide construct is a bispecific engager molecule comprising a single chain variable fragment (scFv) activation domain that binds CD3 and an scFv antigen-recognition domain that binds CLL-1. As defined in claim 2, the polypeptide construct is a bispecific engager molecule which is secreted from the engineered lymphocytes. As defined in claim 1, The antigen-recognition domain is an antibody fragment that binds CLL-1. As defined in claim 1, the antibody fragment that binds CLL-1 is a single chain variable fragment (scFv). The activation domain may be a molecular moiety that interacts with T cell receptor (TCR) and induces an immunomodulatory signal. As defined in claim 1, the activation domain is an antibody fragment that binds CD3. The antibody fragment that binds CD3 may be a single chain variable fragment (scFv). As defined in claim 1, the polypeptide construct is a single polypeptide bispecific engager molecule comprising an scFv activation domain that binds CD3 and an scFv antigen-recognition domain that binds CLL-1. As defined in claim 3, the engineered lymphocytes are engineered T-cells, engineered NK cells, or engineered NKT cells. As defined in claim 3, the method further comprises obtaining lymphocytes from the subject, genetically engineering the lymphocytes to express the polypeptide construct, and culturing the resulting engineered lymphocytes.

Provided herein are engineered lymphocytes as defined in claim 4. As defined in claim 5, the engineered lymphocyte expresses the bispecific engager molecule from the polynucleotide and the bispecific engager molecule is secreted from the engineered lymphocyte. As defined in claim 5, he engineered lymphocyte is a T cell. As defined in claim 5, the engineered lymphocyte is an NK cell.

The invention claims engineered lymphocytes as defined in the claims, including engineered lymphocytes of one of claims 4-5 for use in the treatment of a subject as defined in claim 6. As defined in claim 7, the subject suffers from cancer. As defined in claim 7, the subject suffers from leukemia. As defined in claim 7, the subject suffers from acute myeloid leukemia (AML). As defined in claim 8, the engineered lymphocyte expresses and secretes the bispecific engager molecule within the subject. As defined in claim 8, the bispecific engager molecule binds to malignant cells displaying CLL-1 and T cells, thereby activating the T cells to attack the malignant cells displaying CLL-1.

### BRIEF DESCRIPTION OF THE DRAWINGS

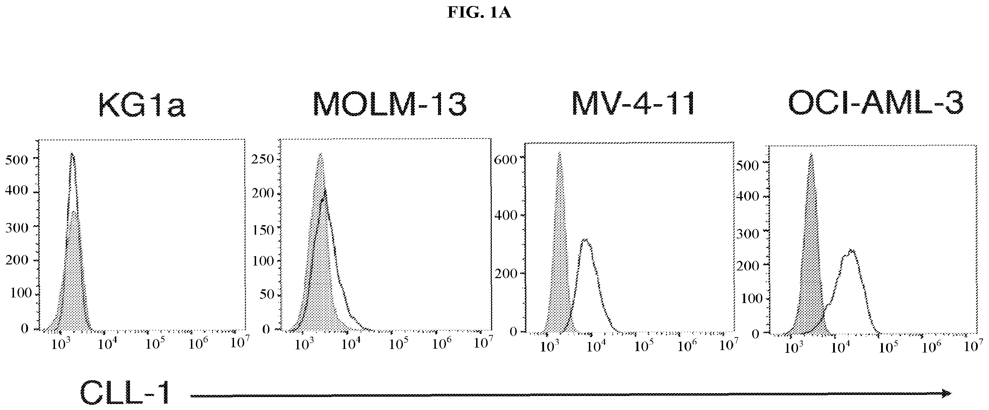

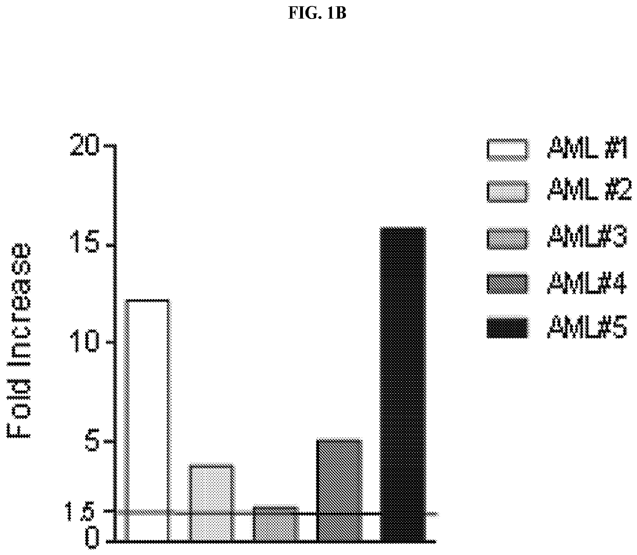

FIG. 1. Demonstration that CLL-1 is expressed on a range of human myeloid leukemia cell lines and primary acute myeloid leukemia samples. (A) FACS analysis of established KG1a, MOLM-13, MV-4-11, and OCI-AML-3, human myeloid leukemia cell lines. Anti-human CLL-1-FITC (clone: 50C1, BD Pharmingen) and isotype (shaded, mouse IgG1k, eBioscience)). (B) Primary human acute myeloid leukemia samples #1-5 express CLL-1. Anti-human CLL-1-FITC, fold increase of MFI over isotype.

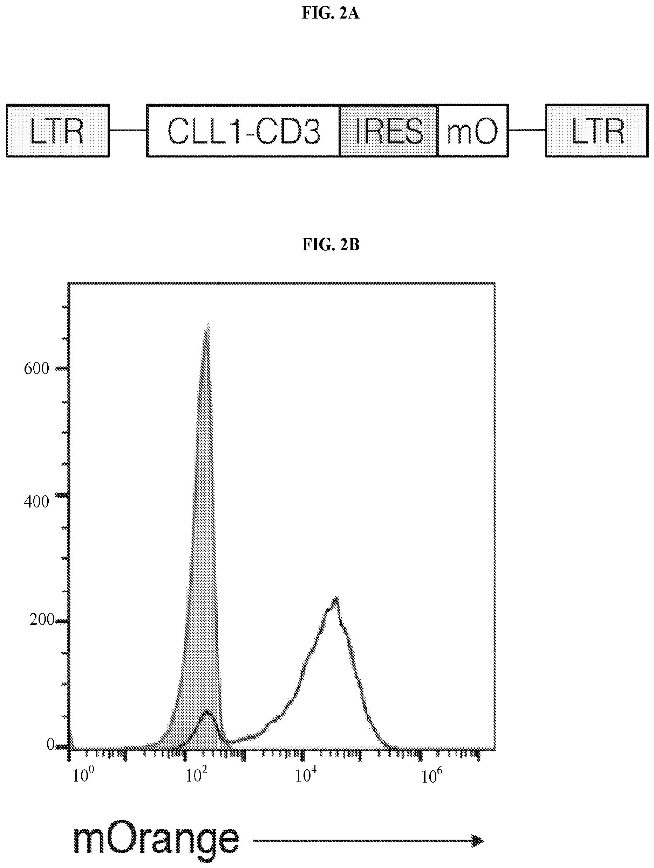

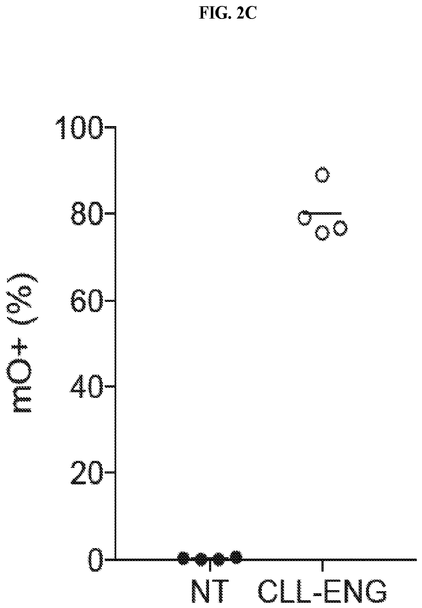

FIG. 2. Demonstration that human T cells can be transduced to express bispecific engager specific for CLL-1 and CD3ε (CLL1-ENG). (A) Schematic of retroviral vector expressing CLL1-ENG. IRES: Internal ribosomal entry site. mO: mOrange fluorescent protein. (B) FACS analysis for mOrange expression following representative transduction. (C) FACS analysis for mOrange expression following CLL1-ENG transduction of T cells isolated from 3 healthy donors. Non-transduced T cells used as control. (mean transduction efficiency: 81%, range: 76-89%)

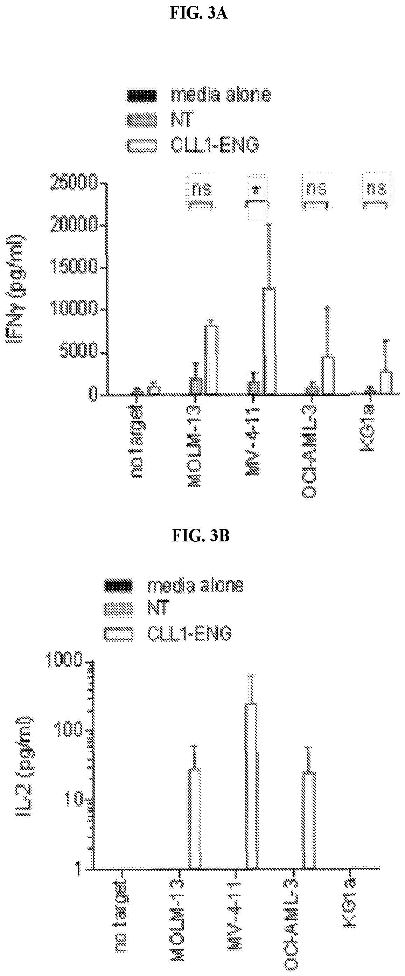

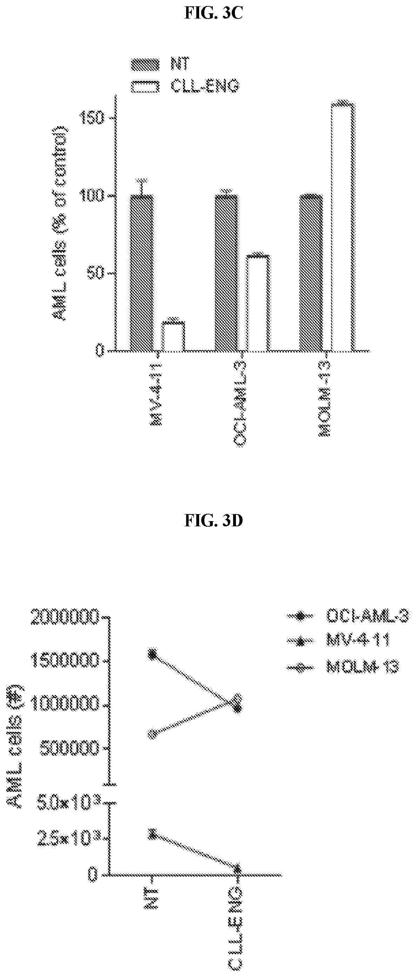

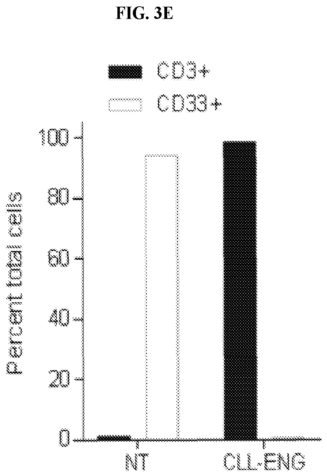

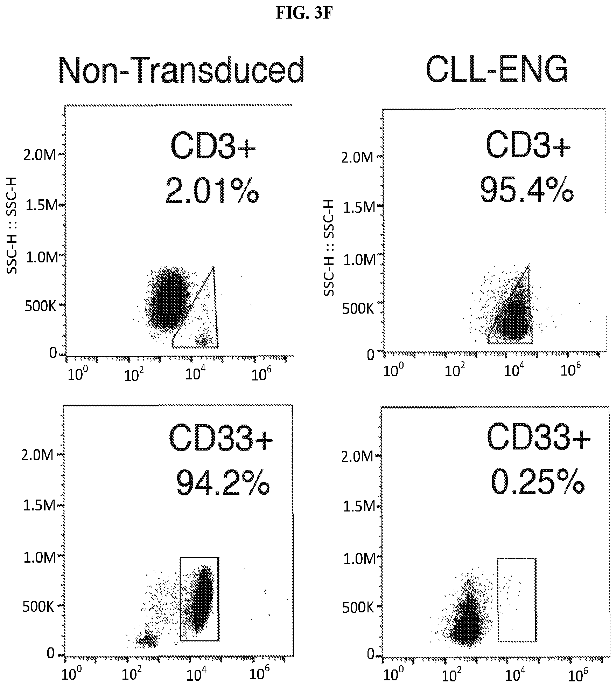

FIG. 3. Demonstration of CLL1-ENG T cell activation and functional activity versus CLL-1-positive target cells. (A) CLL1+ AML cell lines (MOLM-13, MV-4-11, OCI-AML-3) and CLL- AML cell line (KG1a) were cultured with non-transduced or CLL1-ENG T cells at an effector to target ratio of 1:1. 24 hours after culture, supernatant was isolated and tested for the presence of Interferon gamma (IFNγ, n=3 T cell donors). AML cells without T cells and T cells without target cells were included as control populations. (B) Co-culture experiments conducted and supernatant tested for Interleukin-2 (IL-2, n=3 T cell donors). (C) AML cell lines modified to stably express GFP.ffLuc were cultured with non-transduced or CLL1-ENG T cells at an effector:target ratio of 1:10. 7 days following induction of co-culture, presence of live AML cells was measured with luciferase assay. (D) CLL-1+ MOLM-13.ffLuc AML cell line plated with non-transduced or CLL1-ENG T cells at E:T ratio of 1:10. 24 hours and 3 days after plating, presence of live cells measured with luciferase assay. (E) FACS plots of MV-4-11 and T cell co-culture 8 days after plating.

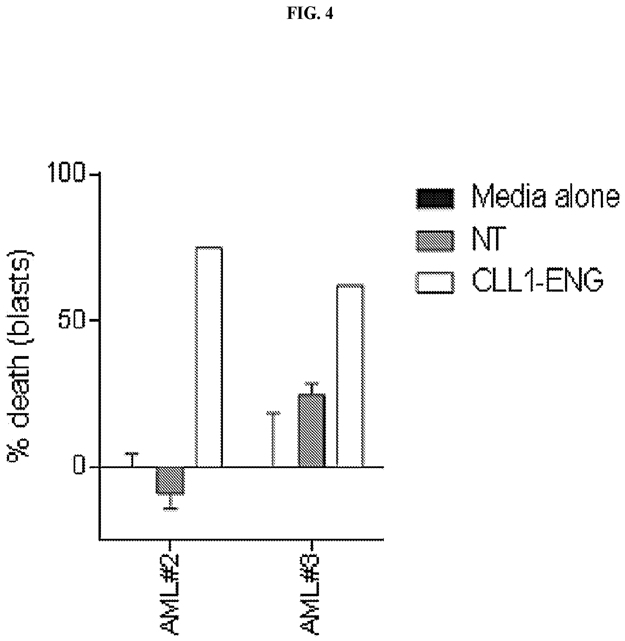

FIG. 4. Demonstration of CLL1-ENG activation of autologous primary T cells versus acute myeloid leukemia. (A) Peripheral blood samples (including primary leukemia and CD3+ T cells) were plated in the bottom of a tissue culture plate. CLL1-ENG T cells were plated in a 0.4 uM pore transwell. Seven days after plating, blast percentage was measured with FACS analysis (CD45med, SShi, and CD117+). No T cells and non-transduced T cells were plated in transwell as a negative controls. Blast percentage normalized to condition with no T cells in transwell.

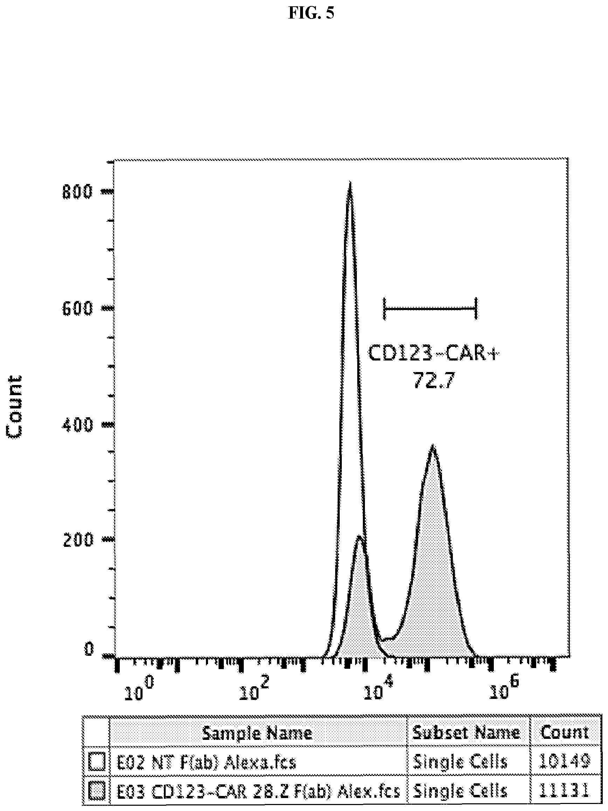

FIG. 5. Demonstration of CD123-CAR expression. FACS analysis performed following retroviral transduction of activated T-cells with retroviral construct containing CD123 scFv, CD28 co-stimulatory region, and CD3ζ intracellular signaling domain.

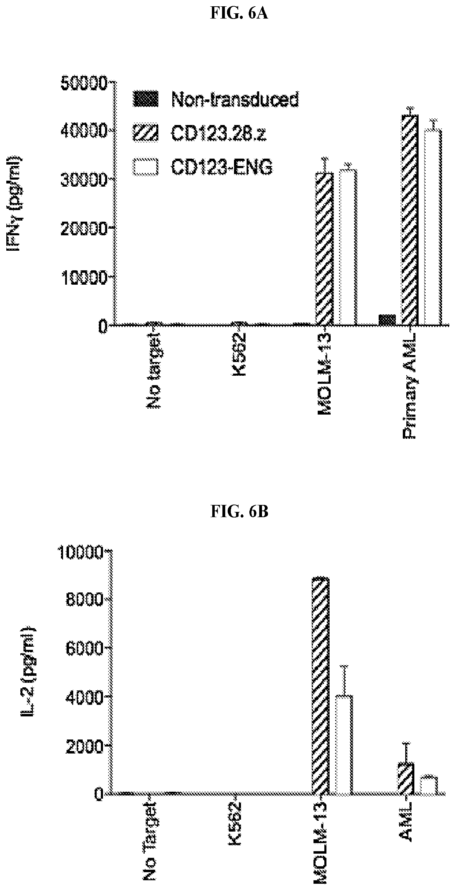

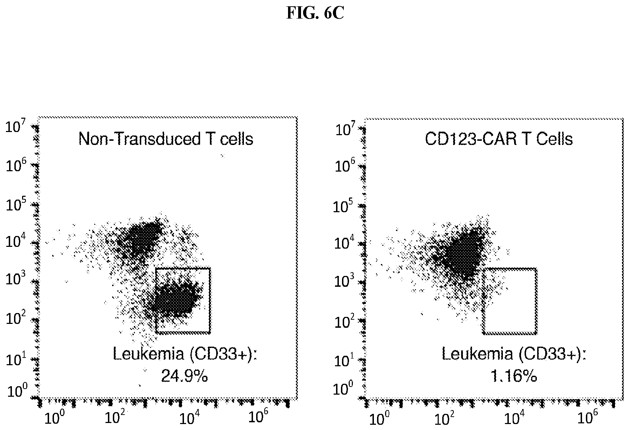

FIG. 6. Demonstration of CD123-CAR T cell activation and cytotoxicity versus CD123+ target cells. (A) CD123+ AML cell lines (MOLM-13, primary AML) and CD123-AML cell line (K562) were cultured with non-transduced or CD123-CAR T cells at an effector to target ratio of 1:1. 24 hours after culture, supernatant was isolated and tested for the presence of Interferon gamma (IFNγ, n=1 T cell donor). T cells without target cells were included as control populations. (B) Co-culture experiments conducted and supernatant tested for Interleukin-2 (IL-2, n=1 T cell donors). (C) MOLM-13 CD123+CD33+ cell line cultured with non-transduced or CD123-CAR T cells at an effector:target ratio of 1:1. 24 hours following induction of co-culture, presence of live AML cells was measured with FACS analysis for cells expressing the myeloid antigen CD33.

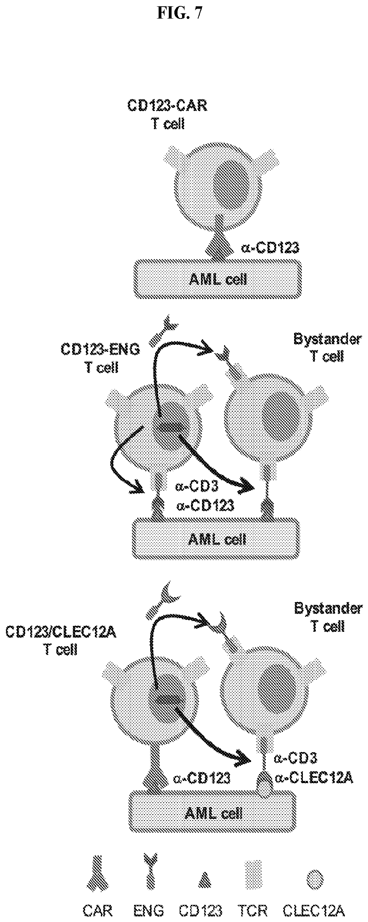

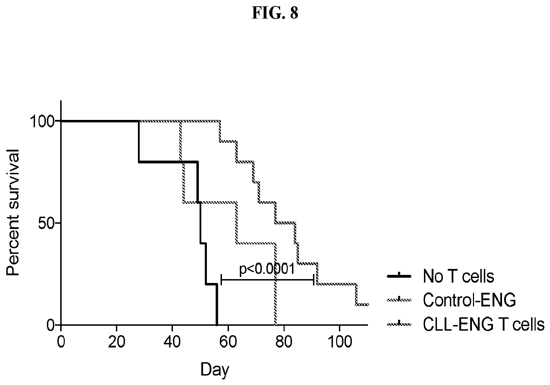

FIG. 7. Utility of CD123 and CLL-1 targeting. A. Antigen expression on AML cell lines and primary AML samples. Fold increase as compared to isotype control. B. Representative FACS plot of primary sample AML#3. C. Gating strategy to identify primary blasts. FIG. 8. A schematic depicting the immunomodulatory action of exemplary (A) CD123-CAR-T cells, (B) CD123-ENG T cells, and (C) combinatorial CD123-CAR/CLL1-ENG T cells.
FIG. 8. CLL-ENG activity in vivo. NOD.Cg-Prkdc^{scid}Il2rg^{tm1Wjl}/SzJ mice (NSG, The Jackson Laboratory, Sacramento, CA) were administered an i.v. tail vein injection of 1e6 MV-4-11.ffLuc leukemia cells on D0. On D7 they were given no treatment, 10e6 control ENG-T cells, or 10e6 CLL-ENG T cells i.v. via tail vein. Leukemia progression was monitored with detection of whole bioluminescence per mouse and analyzed with IVIS software (Perkin Elmer, Waltham, MA). Median survival of mice without treatment: 50 days (n=5), with control-ENG T cells: 63 days (n=5), CLL-ENG T cells: 80.5 days (n=10).

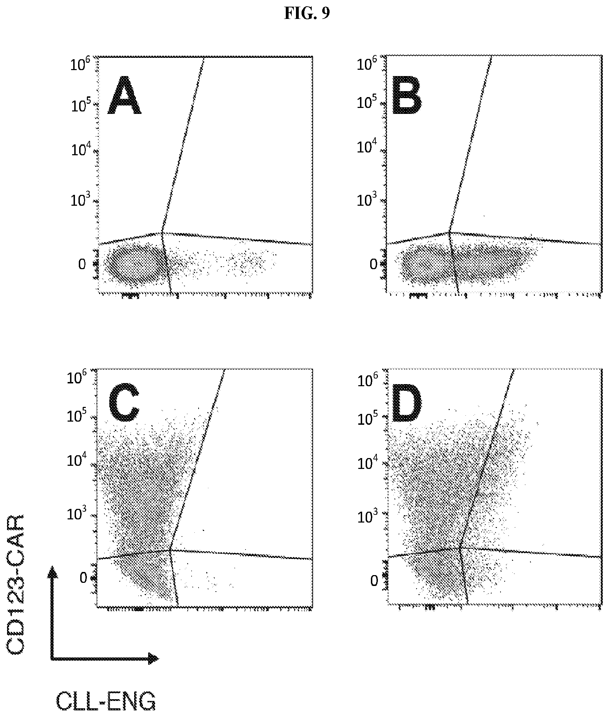

FIG. 9A-D. Co-expression of CLL-ENG and CD123-CAR in T cells. T cells were transduced with retroviral vectors containing either CLL-ENG paired with the flurophore mOrange, or a CD123 chimeric receptor paired with the fluorophore ZsGreen. Successful genetic modification was evaluated with FACS analysis and detection of mOrange and/or ZsGreen. A. Unmodified T cells, B. CLL-ENG T cells, C. CD123-CAR T cells, D. CLL-ENG.CD123-CAR T cells. Red circle represents dual modified cells

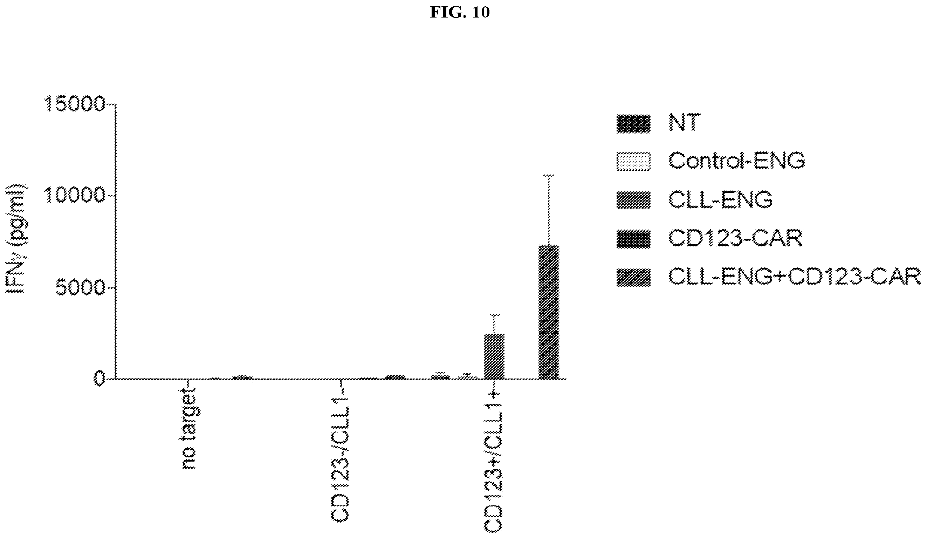

FIG. 10. Enhanced activation with dual T cell modification. Modified T cells and target (CD123-negative/CLL1-negative or CD123positive/CLL1-positive) were plated in co-culture with unmodified, control-ENG, CLL-ENG, CD123-CAR, or CLL-ENG/CD123-CAR T cells. After 24 hours of culture, supernatant was harvested and assessed for the presence of Interferon-gamma as a marker of T cell activation. Experiment performed in technical duplicate with two unique T cell donors.

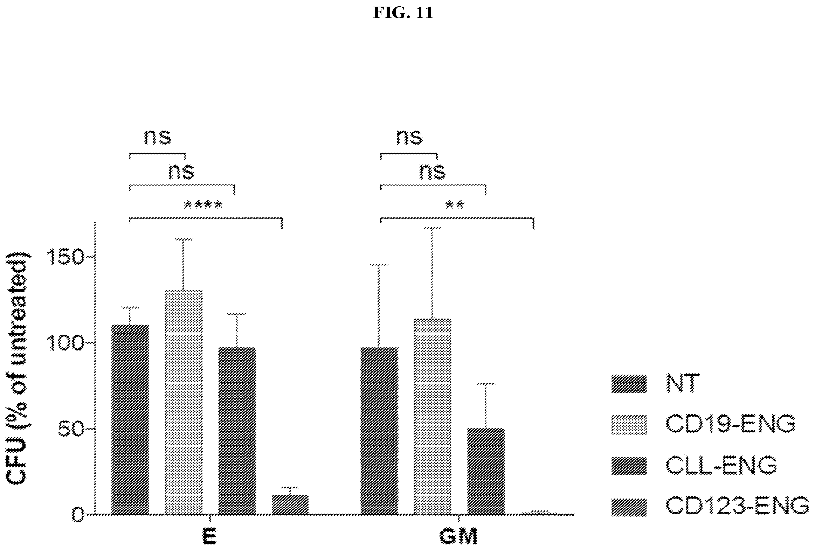

FIG. 11. Lack of toxicity to stem and progenitor hematopoietic cells by CLL1-targeting. Bone marrow mononuclear cells (BMMC) were isolated from healthy donor bone marrow via a density gradient. BMMC were plated with T cells (unmodified (NT), CD19-ENG, CLL-ENG, and CD123-ENG) at a 5:1 T cell to BMMC ratio and incubated for six hours at 37 degrees Celcius. After incubation, cells were plated in semisolid media containing growth factors (MethoCult H4434, Stem Cell Technologies, Cambridge, MA) and incubated for 12 days. Colonies were counted manually, identified as CFU-E or CFU-GM and count was then normalized to that of identical conditions without T cells present. (n=2 unique bone marrow donors and n=2 T cell donors. Each bone marrow challenged with each T cell donor, ns-non-significant difference, ****p<0.0001, **p<0.01).

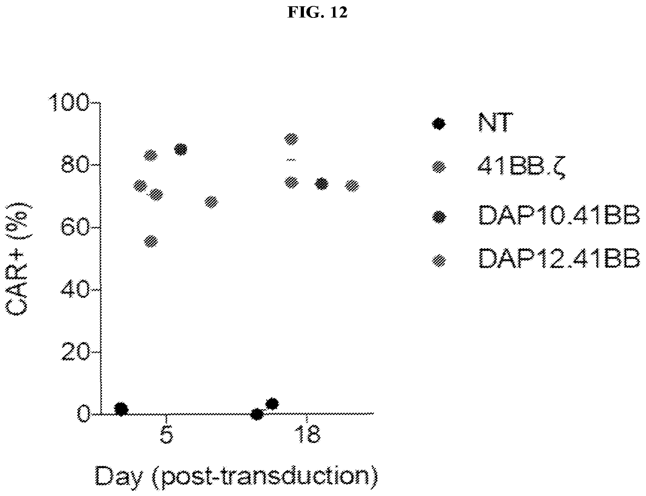

FIG. 12. Demonstration of CAR-NK modification. Healthy donor NK cells were selected from peripheral blood mononuclear cells and transduced with retroviral vectors encoding chimeric receptors. Receptors contain an ectodomain to bind CD123 and an endodomain consisting of a combination of: TCRζ, DAP10, DAP12, 41BB, and/or CD28. CAR expression measured on D5 and D18 post-transduction with FACS analysis. Mean %CAR+ cells D5, D18: 41BB.ζ - 70.6, 81.3; CD28.ζ - 71 (D5 only); DAP10.41BB - 85, 73; DAP12.41BB - 68.1, 73.2.

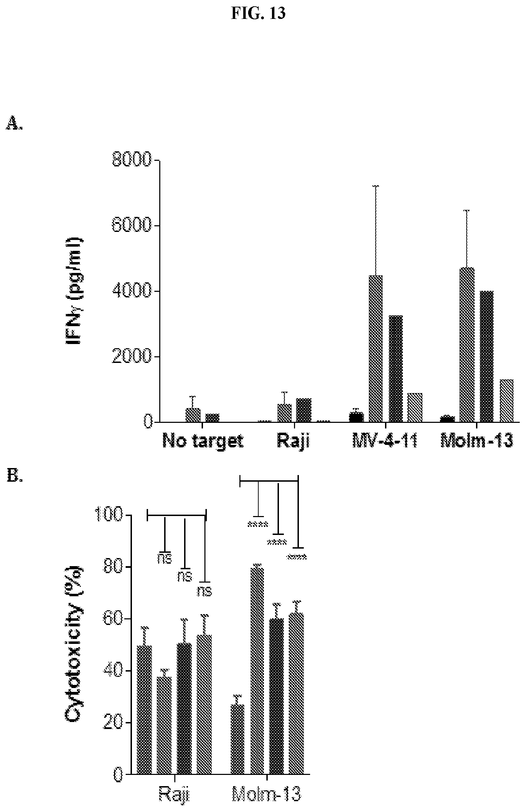

FIG. 13A-B. Demonstration of specific NK- cell activation. A. NK cells and target (Raji (CD123-negative, MV4;11 and Molm-13 (CD123-positive)) were plated in co-culture with unmodified, 41BB.ζ, DAP10.41BB, or DAP12.41BB CAR-NK cells. After 24 hours of culture, supernatant was harvested and assessed for the presence of Interferon-gamma as a marker of NK cell activation. Experiment performed in technical duplicate with 1-3 unique NK cell donors. B. NK cells and target (stably expressing firefly luciferase) were plated at a 1:2 effector:target ratio and cultured for 18 hours. BLI was then measured as a correlate to living cells in culture. Cytoxicity was then calculated as a comparison between cells identically treated without NK cells present. No difference was seen in cytoxicity of unmodified NK or NK-CAR for Raji (CD123-negative). Significantly increased killing (p<0.0001) was evident when comparing unmodified to CAR-NK for Molm-13 (CD123-positive).

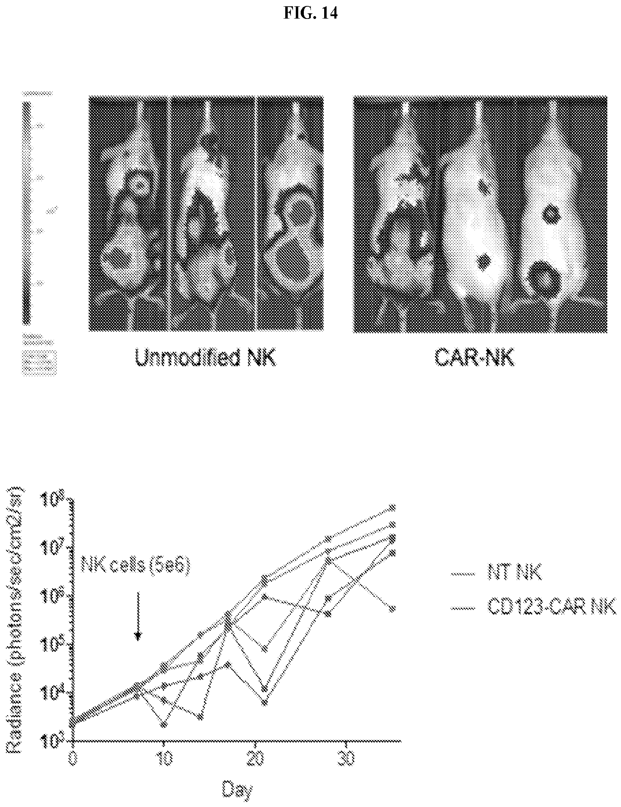

FIG. 14. Demonstration of *in vivo* activation of NK-CAR. NOD.Cg-Prkdc^{scid}Il2rg^{tm1Wjl}/SzJ mice (NSG, The Jackson Laboratory, Sacramento, CA) were administered an i.v. tail vein injection of 1e6 MV-4-11.ffLuc leukemia cells on D0. On D7 they were given no treatment or 5e6 CD123-CAR NK cells. Leukemia progression was monitored with detection of whole bioluminescence per mouse and analyzed with IVIS software (Perkin Elmer, Waltham, MA). Representative images of mice on D28 post-leukemia injection are shown. Line graph is representative of bioluminescence measured per mouse on indicated day post-leukemia injection. BLI displayed as radiance (photons/sec/cm²/sr).

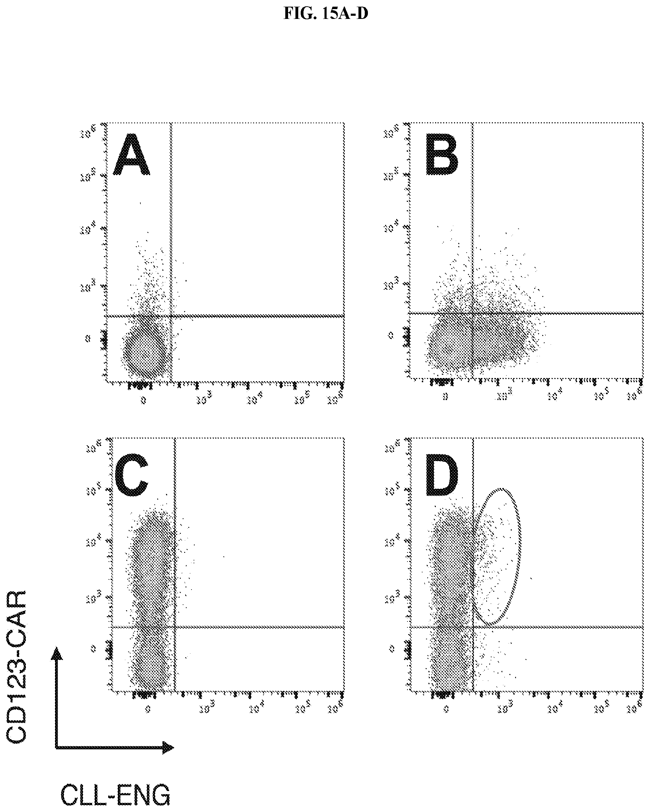

FIG. 15A-D. Demonstration of dual-NK modification. NK cells were transduced with retroviral vectors containing either CLL-ENG paired with the fluorophore mOrange, or a CD123 chimeric receptor. Successful genetic modification was evaluated with FACS analysis and detection of mOrange and/or CAR expression. Unmodified NK cells, B. CLL-ENG NK cells, C. CD123-CAR NK cells, D. CLL-ENG.CD123-CAR NK cells. Circle represents dual modified cells.

### DEFINITIONS

Although any methods and materials similar or equivalent to those described herein can be used in the practice or testing of the disclosure described herein, some preferred methods, compositions, devices, and materials are described herein. However, before the present materials and methods are described, it is to be understood that there is no limitation to the particular molecules, compositions, methodologies or protocols herein described, as these may vary in accordance with routine experimentation and optimization. It is also to be understood that the terminology used in the description is for the purpose of describing the particular versions only.

Unless otherwise defined, all technical and scientific terms used herein have the same meaning as commonly understood by one of ordinary skill in the art to which this invention belongs. However, in case of conflict, the present specification, including definitions, will control. Accordingly, in the context described herein, the following definitions apply.

As used herein and in the appended claims, the singular forms "a", "an" and "the" include plural reference unless the context clearly dictates otherwise. Thus, for example, reference to "a bispecific engager molecule" is a reference to one or more bispecific engager molecules and equivalents thereof known to those skilled in the art, and so forth.

As used herein, the term "comprise" and linguistic variations thereof denote the presence of recited feature(s), element(s), method step(s), etc. without the exclusion of the presence of additional feature(s), element(s), method step(s), etc. Conversely, the term "consisting of" and linguistic variations thereof, denotes the presence of recited feature(s), element(s), method step(s), etc. and excludes any unrecited feature(s), element(s), method step(s), etc., except for ordinarily-associated impurities. The phrase "consisting essentially of" denotes the recited feature(s), element(s), method step(s), etc. and any additional feature(s), element(s), method step(s), etc. that do not materially affect the basic nature of the composition, system, or method. Many examples herein are described using open "comprising" language. Such examples encompass multiple closed "consisting of" and/or "consisting essentially of" examples, which may alternatively be used or described using such language.

As used herein, the term "engager" ("ENG") refers to a molecule that is secreted from a cell and activates immune cells with which it interacts. The engager activates specific immune cells according to the domains present in the engager. Illustrative examples of cells that secrete engagers, but are not limited to, include T-cells, NK cells, NKT cells, CAR T-cells, mesenchymal stem cells (MSCs), neuronal stem cells, hematopoietic stem cells, or a mixture thereof, in some cases.

As used herein, the term "bispecific" refers to any molecule or molecular complex that has two different binding specificities. The molecule or molecular complex may comprise two separate binding domains, each with the same specificity ("homobispecific") or with specificity for different molecular entities (e.g., antigens) ("heterobispecific").

As used herein, the term "antigen-recognition domain" refers to a molecular moiety (e.g. part of an engager molecule) that recognizes an antigen. Antigens can be of any nature including, but not limited to, proteins, carbohydrates, and/or synthetic molecules.

As used herein, the term "activation domain" refers to a molecular moiety (e.g. part of an engager molecule) that interacts with immune cells (e.g., T cell receptor (TCR)) and induces a positive or negative immunomodulatory signal. Illustrative examples of positive immunomodulatory signals include signals that induce cell proliferation, cytokine secretion, or cytolytic activity. Illustrative examples of negative immunomodulatory signals include signals that inhibit cell proliferation, inhibit the secretion of immunosuppressive factors, or induce cell death.

As used herein, the term "native immune cell" refers to an immune cell that naturally occurs in the immune system of a subject. Illustrative examples include, but are not limited to, T-cells, NK cells, NKT cells, B cells, and dendritic cells.

As used herein, the term "engineered immune cell" refers to an immune cell (e.g., T-cell, NK cell, NKT cell, B cell, dendritic cell, etc.) that is genetically modified.

As used herein, the term "co-stimulatory domain" or "co-stimulatory signaling domain" refers to an intracellular signaling domain of a co-stimulatory molecule. In particular aspects, it refers to a domain that provides additional signals to the immune cell in conjunction with an activation domain. Co-stimulatory molecules are cell surface molecules other than antigen receptors or Fc receptors that provide a second signal required for efficient activation and function of T lymphocytes upon binding to antigen. Illustrative examples of such co-stimulatory molecules include CD27, CD28, 4-1BB (CD137), OX40 (CD134), CD30, CD40, ICOS (CD278), LFA-1, CD2, CD7, LIGHT, NKD2C, CD70, CD80, CD86, and CD83.

The term "chimeric antigen receptor" ("CAR") refers to a recombinant polypeptide construct comprising at least an extracellular antigen-recognition domain, a transmembrane domain and an intracellular signaling domain. Upon binding to their target (e.g., displayed on a cancer cell), CARs typically modify the immune response of the immune cells they are displayed upon.

As used herein, the term "intracellular signaling domain," when used in reference to a cell surface receptor or a CAR, is a moiety responsible for activation of at least one function of the cell upon which the receptor or CAR is displayed. The term "effector function" refers to a specialized function of a cell. For example, effector function of a T cell includes cytolytic activity or helper activity including the secretion of cytokines. Thus the term "intracellular signaling domain" refers to the portion of a protein which transduces the effector function signal and directs the cell to perform a specialized function. To the extent that a truncated portion or variant of a native intracellular signaling domain is active, such a polypeptide may be used in place of the full native chain, as long as it transduces the effector function signal. The term intracellular signaling domain includes any truncated or variant portion of a polypeptide sequence sufficient to transduce the effector function signal. Examples of intracellular signaling domains include the cytoplasmic sequences of the T cell receptor (TCR) and co-receptors that act in concert to initiate signal transduction following antigen receptor engagement, as well as any derivative or variant of these sequences and any synthetic sequence that has the same functional capability. Cytoplasmic signaling sequences that act in a stimulatory manner comprise signaling motifs which are known as immunoreceptor tyrosine-based activation motifs (ITAMs). Examples of ITAM containing cytoplasmic signaling sequences include those derived from TCR zeta, FcR gamma, FcR beta, CD3 gamma, CD3 delta, CD3 epsilon, CD3 zeta, CD5, CD22, CD79a, CD79b, and CD66d.

As used herein, the term "transmembrane domain," when used in reference to a cell surface receptor or a CAR, is a moiety that spans the plasma membrane of the cell and is connected to both the intracellular signaling domain and the extracellular antigen-recognition domain. A transmembrane domain may be derived either from a natural or from a synthetic source. Where the source is natural, the domain may be derived from any membrane-bound or transmembrane protein, for example, the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137, CD154, etc. Alternatively the transmembrane domain may be synthetic, in which case it will comprise predominantly hydrophobic residues such as leucine and valine. A triplet of phenylalanine, tryptophan and valine may be found at each end of a synthetic transmembrane domain. Optionally, a short oligo- or polypeptide linker, preferably between 2 and 10 amino acids in length may form the linkage between the transmembrane domain and the intracellular signaling domain. A glycine-serine doublet provides a particularly suitable linker.

As used herein, an "immune response" refers to the action of a cell of the immune system (e.g., T lymphocytes, B lymphocytes, natural killer (NK) cells, macrophages, eosinophils, mast cells, dendritic cells, neutrophils, etc.) and soluble macromolecules produced by any of these cells or the liver (e.g., antibodies, cytokines, and complement) that results in selective targeting, binding to, damage to, destruction of, and/or elimination from a subject of invading pathogens, cells or tissues infected with pathogens, or cancerous cells or other abnormal/diseased-associated cells.

As used herein, the term "immunotherapy" refers to the treatment or prevention of a disease or condition by a method comprising inducing, enhancing, suppressing or otherwise modifying an immune response.

As used herein, the term "adoptive cell transfer" ("ACT") is the transfer of cells into a patient. The cells may have originated from the patient or from another individual or cell line. The cells are most commonly derived from the immune system, with the goal of improving immune functionality or eliciting a desired immune response. Cells may be extracted from a subject, genetically modified (e.g., to express a desired construct (e.g., CAR or endanger molecule)), cultured in vitro, and returned to the subject.

As used herein, the term "antibody" refers to a whole antibody molecule or a fragment thereof (e.g., fragments such as scFv, Fab, Fab', and F(ab')₂), unless specified otherwise; an antibody may be a polyclonal or monoclonal antibody, a chimeric antibody, a humanized antibody, a human antibody, etc.

A native antibody typically has a tetrameric structure. A tetramer typically comprises two identical pairs of polypeptide chains, each pair having one light chain (e.g, about 25 kDa) and one heavy chain (e.g., about 50-70 kDa). In a native antibody, a heavy chain comprises a variable region, V_{H}, and three constant regions, C_{H1}, C_{H2}, and C_{H3}. The V_{H} domain is at the amino-terminus of the heavy chain, and the C_{H3} domain is at the carboxy-terminus. In a native antibody, a light chain comprises a variable region, V_{L}, and a constant region, C_{L}. The variable region of the light chain is at the amino-terminus of the light chain. In a native antibody, the variable regions of each light/heavy chain pair typically form the antigen binding site. The constant regions are typically responsible for effector function.

In a native antibody, the variable regions typically exhibit the same general structure in which relatively conserved framework regions (FRs) are joined by three hypervariable regions, also called complementarity determining regions (CDRs). The CDRs from the two chains of each pair typically are aligned by the framework regions, which may enable binding to a specific epitope. From N-terminus to C-terminus, both light and heavy chain variable regions typically comprise the domains FR1, CDR1, FR2, CDR2, FR3, CDR3 and FR4. The CDRs on the heavy chain are referred to as H1, H2, and H3, while the CDRs on the light chain are referred to as L1, L2, and L3. Typically, CDR3 is the greatest source of molecular diversity within the antigen-binding site. The assignment of amino acids to each domain is typically in accordance with the definitions of Kabat et al. (1991) Sequences of Proteins of Immunological Interest (National Institutes of Health, Publication No. 91-3242, vols. 1-3, Bethesda, Md.); Chothia, C., and Lesk, A. M. (1987) J. Mol. Biol. 196:901-917; or Chothia, C. et al. Nature 342:878-883 (1989). In the present application, the term "CDR" refers to a CDR from either the light or heavy chain, unless otherwise specified.

As used herein, the term "heavy chain" refers to a polypeptide comprising sufficient heavy chain variable region sequence to confer antigen specificity either alone or in combination with a light chain.

As used herein, the term "light chain" refers to a polypeptide comprising sufficient light chain variable region sequence to confer antigen specificity either alone or in combination with a heavy chain.

As used herein, when an antibody or other entity "specifically recognizes" or "specifically binds" an antigen or epitope, it preferentially recognizes the antigen in a complex mixture of proteins and/or macromolecules, and binds the antigen or epitope with affinity which is substantially higher than to other entities not displaying the antigen or epitope. In this regard, "affinity which is substantially higher" means affinity that is high enough to enable detection of an antigen or epitope which is distinguished from entities using a desired assay or measurement apparatus. Typically, it means binding affinity having a binding constant (Kₐ) of at least 10⁷ M⁻¹ (e.g., >10⁷ M⁻¹, >10⁸ M⁻¹, >10⁹ M⁻¹, >10¹⁰ M⁻¹, >10¹¹ M⁻¹, >10¹² M⁻¹, >10¹³ M⁻¹, etc.). An antibody may be capable of binding different antigens so long as the different antigens comprise that particular epitope. In certain instances, for example, homologous proteins from different species may comprise the same epitope.

As used herein, the term "antibody fragment" refers to a portion of a full-length antibody, including at least a portion antigen binding region or a variable region. Antibody fragments include, but are not limited to, Fab, Fab', F(ab')₂, Fv, scFv, Fd, diabodies, and other antibody fragments that retain at least a portion of the variable region of an intact antibody. See, e.g., Hudson et al. (2003) Nat. Med. 9:129-134. Antibody fragments may be produced by enzymatic or chemical cleavage of intact antibodies (e.g., papain digestion and pepsin digestion of antibody) produced by recombinant DNA techniques, or chemical polypeptide synthesis.

For example, a "Fab" fragment comprises one light chain and the C_{H1} and variable region of one heavy chain. The heavy chain of a Fab molecule cannot form a disulfide bond with another heavy chain molecule. A "Fab'" fragment comprises one light chain and one heavy chain that comprises additional constant region, extending between the C_{H1} and C_{H2} domains. An interchain disulfide bond can be formed between two heavy chains of a Fab' fragment to form a "F(ab')₂" molecule.

An "Fv" fragment comprises the variable regions from both the heavy and light chains, but lacks the constant regions. A single-chain Fv (scFv) fragment comprises heavy and light chain variable regions connected by a flexible linker to form a single polypeptide chain with an antigen-binding region. Exemplary single chain antibodies are discussed in detail in WO 88/01649 and U.S. Pat. Nos. 4,946,778 and 5,260,203. In certain instances, a single variable region (e.g., a heavy chain variable region or a light chain variable region) may have the ability to recognize and bind antigen.

Other antibody fragments will be understood by skilled artisans.

As used herein, the term "single-chain bispecific antibody construct" refers to a polypeptide construct comprising two antibody-derived binding domains. The two antibody-derived binding domains may be an antigen-recognition domain and an activation domain. The binding domains may comprise variable regions (or parts thereof) of an antibody, antibody fragment or derivative thereof, capable of specifically binding to/interacting with a target antigen (e.g., CLL-1) or an activation molecule (e.g., human CD3 antigen). A part of a variable region may comprise at least one CDR ("Complementary determining region"), such as at least a CDR1, CDR2, or CDR3 region. The two domains/regions in the single chain antibody construct are preferably covalently connected to one another as a single chain. Illustrative examples of bispecific single chain molecules are known in the art and are described in WO 99/54440; Mack, J. Immunol. (1997), 158, 3965-3970; Mack, PNAS, (1995), 92, 7021-7025; Kufer, Cancer Immunol. Immunother., (1997), 45, 193-197; Loffler, Blood, (2000), 95, 6, 2098-2103; and Bruhl, J. Immunol., (2001), 166, 2420-2426.

As used herein, the terms "administration" and "administering" refer to the act of giving a drug, prodrug, or other agent, or therapeutic to a subject or *in vivo, in vitro,* or *ex vivo* cells, tissues, and organs. Exemplary routes of administration to the human body can be through space under the arachnoid membrane of the brain or spinal cord (intrathecal), the eyes (ophthalmic), mouth (oral), skin (topical or transdermal), nose (nasal), lungs (inhalant), oral mucosa (buccal), ear, rectal, vaginal, by injection (e.g., intravenously, subcutaneously, intratumorally, intraperitoneally, etc.) and the like.

As used herein, the terms "co-administration" and "co-administering" refer to the administration of at least two agent(s) or therapies to a subject. The co-administration of two or more agents or therapies may be concurrent. A first agent/therapy may be administered prior to a second agent/therapy. Those of skill in the art understand that the formulations and/or routes of administration of the various agents or therapies used may vary. The appropriate dosage for co-administration can be readily determined by one skilled in the art. When agents or therapies are co-administered, the respective agents or therapies may be administered at lower dosages than appropriate for their administration alone. Thus, co-administration is especially desirable where the co-administration of the agents or therapies lowers the requisite dosage of a potentially harmful (e.g., toxic) agent(s), and/or when co-administration of two or more agents results in sensitization of a subject to beneficial effects of one of the agents via co-administration of the other agent.

### DETAILED DESCRIPTION

The invention is set out in the appended claims. The embodiments of the description which do not fall within the scope of said claims are provided for illustrative purposes only and do not form part of the present invention. Provided here are compositions comprising engineered lymphocytes that secrete bispecific engager molecules, thereby activating T cells in the local environment to kill target cells. As described herein, engineered lymphocytes selectively target CLL-1 positive leukemic cells, both directly and through activation of a subject's own T cells, while sparing CLL-1 negative cells, such as myeloid progenitor cells.

Lymphocytes may be engineered to express/display chimeric antigen receptors (CARs) that bind to target cells (e.g., via an antigen-recognition domain), thereby activating the engineered T cells (e.g., via an intracellular signaling domain) toward the target cells (Figure 10A). The engineered lymphocytes may display a CAR directed to CLL-1 antigen and/or CD123.

Lymphocytes may be engineered to express/secrete bispecific engager molecules that bind to (i) target cells and (ii) engineered and/or native T cells, and thereby activate T cells toward the target cells (Figure 10B). Engager molecules may target cells displaying a CLL-1 antigen and activate T cells (e.g., engineered and/or native T cells) thereto.

Engineered lymphocytes expressing both a CAR and an engager molecule may be provided (Figure 10C). For example, a lymphocyte may express/display a CAR against a first antigen and express/secrete a bispecific engager molecule against a second antigen and T cell receptor (TCR). The CAR targets the engineered lymphocytes to target cells displaying the first antigen, thereby effecting an immune response by the engineered lymphocyte against the target cell, and localizing the engineered lymphocytes at a treatment site. The engager molecule binds target cells displaying the second antigen (e.g., via its antigen-recognition domain), and also binds T cells (e.g., via its activation domain), thereby effecting an immune response by the engineered lymphocytes as well as native T cells. Localization of the engineered lymphocytes by the CAR, results in release of engager molecules at the treatment cite and concentration of the immune response by the native T cells. The first and second antigens may be the same or different antigens. The first and second antigens may be both displayed on the surface of the target cells.

Disclosure herein relates to a strategy of adoptive cell transfer of lymphocytes transduced to express a chimeric antigen receptor (CAR), an engager molecule (ENG), or both, for the treatment of cancer (e.g., AML) or other diseases (e.g., infection) in a subject.

Provided herein are compositions comprising engager molecules (e.g., a bispecific engager molecule), wherein the engager molecule comprises (i) a domain that binds to an antigen on an immune cell surface (e.g., native or engineered immune cell surface) and (ii) an domain that binds to a target cell antigen (e.g., an antigen expressed on the surface of tumor cell, cancer cell, or other disease-related cell).

Engager cells (e.g., cells that express and bispecific engager molecules) are provided. Methods are provided comprising administering engager cells to a subject provide therapy (e.g., cancer immunotherapy).

Cells (e.g., immune cells) may be genetically modified to express engager molecules comprising at least (i) an antigen-recognition domain and (ii) an activation domain. Engager molecules expressed by engineered cells may further comprise one or more accessory domains, such as a cytokine domain, costimulatory domain, a domain for inhibition of negative regulatory molecules of T-cell activation, etc. An antigen-recognition domain, an activation domain, and/or any accessory domains present in an engager molecule may be directly linked or are linked by a linker domain. The linker domain may have contained within it a functional moiety, for example, the activation domain of a human cytokine.

The antigen-recognition domain of an engager molecule may bind to one or more molecules present in and/or on target cells or that are secreted by target cells. Target cells may be cancer cells, including at least hematological tumor cells (e.g., hematological malignancies derived from myeloid cell lines). Engager molecules bound to an antigen on a target molecule, are capable of activating immune cells (e.g., engineered or native immune cells) that express/display the molecular determinant recognized by the activation domain of the engager molecule. Engager molecules activate engineered immune cells (e.g., those expressing/secreting engager molecules, those engineered to express other immunotherapeutically-useful agents, etc.) that express/display the molecular determinant recognized by the activation domain. Engager molecules also activate native immune cells (e.g., unmodified immune cells that are native to the subject being treated) that express/display the molecular determinant recognized by the activation domain. Immune cell activation results in a positive or negative signal, depending upon the molecular determinant recognized by the activation domain. Examples of positive signals include signals that induce cell proliferation, cytokine secretion, or cytolytic activity. Examples of negative signals include signals that inhibit-cell proliferation, inhibit the secretion of immunosuppressive factors, or induce cell death. By activating native immune cells, engineered immune cells that secrete engager molecules redirect resident (e.g., naturally endogenous to a specific individual) immune cells to target cells.

Engager molecules may comprise a polypeptide chain comprising an antigen-recognition domain and an activation domain. The antigen-recognition domain and the activation domain may be linked directly. The antigen-recognition domain and the activation domain may be connected by a linker peptide. An engager molecule may be a single-chain bispecific antibody construct. The antigen-recognition domain may be a single chain variable fragment that binds a target cell antigen. The activation domain may be a single chain variable fragment that engages the TCR. Both the activation domain and the antigen-recognition domain may be scFv's and the individual moieties may be arranged and oriented in any suitable manner, for example:
N- V_{H-AD}-L₁-V_{L-AD}-L₂-V_{H-ARD}-L₃-V_{L-ARD} -C;
N- V_{L-AD}-L₁-V_{H-AD}-L₂-V_{H-ARD}-L₃-V_{L-ARD} -C;
N- V_{H}-_{AD}-L₁-V_{L}-_{AD}-L₂-V_{L}-_{ARD}-L₃-V_{H}-_{ARD} -C;
N- V_{L-AD}-L₁-V_{H-AD}-L₂-V_{L-ARD}-L₃-V_{H-ARD} -C;
N- V_{H- ARD}-L₁-V_{L-ARD}-L₂-V_{H-AD}-L₃-V_{L-AD} -C;
N- V_{L}- _{ARD}-L₁-V_{H-ARD}-L₂-V_{H-AD}-L₃-V_{L-AD} -C;
N- V_{H}- _{ARD}-L₁-V_{L-ARD}-L₂-V_{L-AD}-L₃-V_{H-AD} -C;
N- V_{L}- _{ARD}-L₁-V_{H-ARD}-L₂-V_{L-AD}-L₃-V_{H-AD} -C;
wherein N- is the N-terminus; V_{H} is the heavy chain variable region; V_{L} is the light chain variable region; L₁, L₂, and L₃ are linker peptides; -C is the C-terminus; ARD is antigen-recognition domain; and AD is activation domain. L₁ and L₃ may be of appropriate length and sequence to allow the activation scFv and the antigen-binding scFv to each function properly (e.g., bind to TCR and antigen, respectively) individually, and L₂ is or proper length and sequence to allow the activation scFv and antigen-binding scFv to both function (e.g., bind to TCR and antigen) within a single bispecific construct (e.g., engager molecule). L₁, L₂, and/or L₃ may be absent from the above constructs.

An engager molecule may comprise an antigen-recognition domain that binds to an antigen presented on the surface of a diseased cell or cell that is the source of disease. The antigen-recognition domain may bind to an antigen presented on a cancer cell or a tumor cell. Any cancer antigen may be targeted by the engager-expressing T-cells or the corresponding engager molecules thereof. The antigen-recognition domain may bind to an antigen presented on cells of hematopoietic and lymphoid malignancies. The antigen-recognition domain may bind to an antigen presented on cells of myeloid malignancies. The antigen-recognition domain may bind to an antigen presented on cells of acute and chronic myelogenous leukemia, myelodysplastic syndromes and myeloproliferative diseases. The antigen-recognition domain may bind to an antigen presented on cells of myeloid malignancies but not myeloid progenitor cells. The antigen-recognition domain may bind to C-type lectin domain family 12 member A; a human protein which is encoded by the CLL-1 gene; is also referred to as CLEC12A, CLL1, DCAL-2, MICL, and CD371; and is typically referred to herein as CLL-1. Experiments conducted during development of the disclosure herein demonstrate the utility advantageousness of engager molecules comprising CLL-1 antigen-recognition domains and lymphocytes expressing/secreting such engager molecules; however, there is no limitation to CLL-1 antigen-recognition domains.

An engager molecule may comprise an activation domain that allows the engager molecule to bind to an immunoresponsive cell (e.g., T cell). The activation domain may bind to a surface displayed ligand, antigen, receptor, etc. on the engineered lymphocyte from which it was expressed. The activation domain may bind to a surface displayed ligand, antigen, receptor, etc. on native lymphocytes. The activation domain may bind to native lymphocytes but not engineered lymphocytes. The activation domain may bind to native lymphocytes and engineered lymphocytes. The activation domain may be an antibody or antigen-binding fragment thereof (e.g., scFv). Illustrative examples of activation domains include, but are not limited to antibodies, antigen-binding antibody fragments, ligands, peptides, soluble T-cell receptors, or combinations thereof.

The immune cell to which the engager binds may be an unmodified naturally endogenous (to the recipient individual) immune cell (e.g., native lymphocyte), or it may be a genetically modified immune cell (e.g., engineered lymphocyte). Binding of the engager to the target immune cell (e.g., to the TCR) through the activation domain (e.g., via a CD3 antibody of antibody fragment), thereby activates the target immune cell. When the engager is to target NK cells, the activation domain may comprise of an antibody that recognizes, for example, CD16 (such as NM3E2 antibody), or ligands specific for NKG2D (ULBP2), or NKp30 (B7H6). The activation domain may comprise ligands, receptors, peptides, etc.

An engager molecule may comprise an activation domain that targets co-stimulatory molecules such as CD27, CD28, CD134, and CD137. Such engager molecules may be used in concert with other engager molecules (e.g., engager molecule with activation domains that directly stimulate lymphocytes (e.g., that bind the TCR). For example, T-cells are engineered to express a first engager molecule with a CLL-1-specific antigen recognition domain and a CD3-specific activation domain, and another engager with a CD123-specific antigen recognition domain and a CD28-specific activation domain. T-cells would only be fully activated (e.g., activated by both CD3 and CD28 binding) at tumor sites at which both CLL-1 and CD123 antigens are expressed. Co-stimulatory engager molecules may be used in concert with engineered lymphocytes that express/display chimeric antigen receptors. For example, T-cells are engineered to express a chimeric antigen receptor with an CD123-specific antigen recognition domain, and an engager molecule with a CLL-1-specific antigen recognition domain and a CD28-specific activation domain. These engineered T-cells would target any cells displaying a CD123 antigen, but would only be fully activated (at tumor sites at which both CLL-1 and CD123 antigens are expressed. Other combinations of primary (e.g., CD3, TCR, etc.) and co-stimulatory activation domains with engagers, CARs, etc. can be considered.

In addition to activation, linker, and antigen-recognition domains, an engager molecule may comprise one or more additional functional domains (and optionally additional linker domains). Such engager molecules may be trispecific (e.g., capable of binding to three ligands) or multispecifc (e.g., capable of binding to two or more (e.g., 2, 3, 4, 5, 6, 7, 8, or more) ligands) or may be bispecific but further comprise additional functionality (e.g., targeting peptide, therapeutic peptide, luminescence, fluorescence, etc.). Additional functional domains may comprise a cytokine, costimulatory domain, and/or domain for inhibition of negative regulatory molecules of T-cell activation.

As addressed above, engager molecules may comprise one or more linker domains (e.g., between heavy and light variable chains, between activation and antigen-recognition domains, etc.). Linkers that facilitate the formation and activity of scFv constructs (e.g., L₁- and L₃-type linkers, linkers between heavy and light variable chains) are well understood in the art. Such linkers may be about 5 to 50 amino acids in length (e.g., 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or ranges therebetween (e.g., 10-25)). Linkers may be glycine rich (e.g., >20%, >30%, >40%, >50%) to impart flexibility to the linker. Linkers may be serine and/or threonine rich (e.g., >20%, >30%, >40%, >50%) to impart solubility to the linker. Linkers may be of any suitable length and sequence so as to allow the formation of active activation and antigen-recognition domains. Linkers between the activation and antigen-recognition domains (e.g., L₂-type linkers) may comprise similar characteristics (e.g., to L₁- and L₃-type linkers). Linkers between the activation and antigen-recognition domains (e.g., L₂-type linkers) may be of any suitable length and sequence so as to allow the formation of active activation and antigen-recognition domains, without adverse interaction occurring between the domains.

Provided herein are chimeric antigen receptors (CARs) and lymphocytes that are engineered to express/display a desired CAR (e.g., engineered to express a CLL1-targetted CAR alone, engineered to express a CAR and an engager molecule, etc.). A cell may be engineered to stably express an antibody binding domain on its surface, conferring novel antigen specificity (e.g., that is MHC independent). A cell may be engineered to express a CAR that combines an antigen recognition domain of a specific antibody with an intracellular activation domain (e.g., CD3- zeta chain or FcyRI protein) into a single chimeric protein.

A CAR may comprise an extracellular domain having an antigen recognition domain, a transmembrane domain, and a cytoplasmic domain having an intracellular signaling domain. The transmembrane domain that naturally is associated with one of the domains in the CAR may be used. The transmembrane domain may be selected or modified by amino acid substitution to avoid binding of such domains to the transmembrane domains of the same or different surface membrane proteins to minimize interactions with other members of the receptor complex. The transmembrane domain may be the CD8 hinge domain.

With respect to the cytoplasmic domain, a CAR may comprise the CD28 and/or 4-IBB signaling domain by itself or may be combined with any other desired cytoplasmic domain(s) useful in the context of the CAR. The cytoplasmic domain of the CAR may comprise the signaling domain of CD3-zeta. For example, the cytoplasmic domain of the CAR includes but is not limited to CD3-zeta, 4-IBB and CD28 signaling modules and combinations thereof.

Chimeric antigen receptors provided herein comprise an extracellular and intracellular domain. The extracellular domain comprises an antigen recognition domain. The intracellular domain or the cytoplasmic domain may comprise, a costimulatory signaling region and a zeta chain portion. The costimulatory signaling region refers to a portion of the CAR comprising the intracellular domain of a costimulatory molecule. Costimulatory molecules are cell surface molecules other than antigens receptors or their ligands that are required for an efficient response of lymphocytes to antigen.

Between the extracellular domain and the transmembrane domain of the CAR, or between the cytoplasmic domain and the transmembrane domain of the CAR, there may be incorporated a linker domain. A linker domain of a CAR is an oligo- or polypeptide that functions to link the transmembrane domain to, either the extracellular domain or, the cytoplasmic domain in the polypeptide chain. A spacer domain may comprise up to 300 amino acids, preferably 10 to 100 amino acids and most preferably 25 to 50 amino acids.

A CAR may comprise an antigen recognition domain. The choice of antigen recognition domain depends upon the type and number of ligands that define the surface of a target cell. For example, the antigen recognition domain may be chosen to recognize a ligand that acts as a cell surface marker on target cells associated with a particular disease state. Thus examples of cell surface markers that may act as ligands for the antigen moiety domain in the CAR of include those associated with viral, bacterial and parasitic infections, autoimmune disease and cancer cells.

A CARmay target a tumor antigen of interest by displaying an antigen recognition domain that specifically binds to an antigen on a cancer cell. Cancer cell antigens are proteins that are produced by cancer cells that elicit an immune response, particularly T-cell mediated immune responses. The selection of the antigen recognition domain depends on the particular type of cancer to be treated. Cancer antigens are well known in the art. Examples of cancer antigens that may be targeted by CARs in examples herein include CLL-1 and CD123 antigens. The cancer antigen may comprise one or more antigenic cancer epitopes associated with a malignant tumor, metastatic tumor, leukemia (e.g., AML), etc.

The type of cancer antigen targeted herein (e.g., by CAR and/or by bispecific engager) may also be a cancer-specific antigen (CSA) (or tumor-specific antigen (TSA)) or a cancer-associated-antigen (CAA) (or tumor-associated-antigen (TAA)). A CSA is unique to cancer cells and does not occur on other cells in the body (e.g., healthy native cells). A CAA is not unique to a tumor cell and instead is also expressed on normal cells under conditions.

Depending on the desired antigen to be targeted, a CAR is engineered to include the appropriate antigen recognition domain that is specific to the desired antigen target. For example, if CD123 or CLL-1 is the desired antigen that is to be targeted, an antibody (or fragment thereof (e.g., scFv)) for CD123 or CLL-1 is used as the antigen recognition domain for incorporation into the CAR.

The antigen recognition domain of a CAR may target CD123. The antigen recognition domain in the CAR may be anti-CD123 scFV.

The antigen recognition domain of a CAR may target CLL-1. The antigen recognition domain in the CAR may be anti- CLL-1 scFV.

With respect to the transmembrane domain, CARs may be designed to comprise a transmembrane domain that is fused to the extracellular and intracellular domains of the CAR. A transmembrane domain may be a sequence that is naturally associated with one of the other domains in the CAR. The transmembrane domain may be selected or modified by amino acid substitution to avoid interactions with other CAR domains or cell surface components.

A transmembrane domain may be from either a natural or a synthetic source. Where the source is natural, the domain may be derived from any membrane-bound or transmembrane protein. Transmembrane regions of particular use include at least the transmembrane region(s) of known transmembrane proteins, including, but not limited to: the alpha, beta or zeta chain of the T-cell receptor, CD28, CD3 epsilon, CD45, CD4, CD5, CDS, CD9, CD 16, CD22, CD33, CD37, CD64, CD80, CD86, CD 134, CD137, CD 154. Alternatively the transmembrane domain may be synthetic, in which case it will comprise predominantly hydrophobic residues such as leucine and valine. Preferably a triplet of phenylalanine, tryptophan and valine will be found at each end of a synthetic transmembrane domain. Optionally, a short oligo- or polypeptide linker, preferably between 2 and 10 amino acids in length may form the linkage between the transmembrane domain and the cytoplasmic domain of the CAR. A glycine-serine doublet provides a particularly suitable linker.

The transmembrane domain of the CAR may comprise a CD8 hinge domain.

The cytoplasmic domain (a.k.a. intracellular signaling domain, activation domain, etc.) of the CAR is responsible for activation of at least one of the normal effector functions of the immune cell in which the CAR has been placed in. The term "effector function" refers to a specialized function of a cell. Effector function of a T cell, for example, may be cytolytic activity or helper activity including the secretion of cytokines. Thus the term "intracellular signaling domain" refers to the portion of a protein which transduces the effector function signal and directs the cell to perform a specialized function. While the entire intracellular signaling domain of a known protein or protein complex may be employed , it is not necessary to use the entire chain. To the extent that a truncated portion of a known intracellular signaling domain is used, such truncated portion may be used in place of the intact chain as long as it transduces the effector function signal. Examples of intracellular signaling domains for use in the CARs herein include the cytoplasmic sequences of the T cell receptor (TCR) and co-receptors that act in concert to initiate signal transduction following antigen-receptor engagement, as well as any derivative or variant of these sequences and any synthetic sequence that has the same functional capability.

Signals generated through the TCR alone are insufficient for full activation of the T cell; a secondary or co-stimulatory signal is also required for full activation. Thus, T cell activation may be mediated by two distinct classes of cytoplasmic signaling sequence: those that initiate antigen-dependent primary activation through the TCR (primary cytoplasmic signaling sequences) and those that act in an antigen-independent manner to provide a secondary or co-stimulatory signal (secondary cytoplasmic signaling sequences). Primary cytoplasmic signaling sequences regulate primary activation of the TCR complex either in a stimulatory way, or in an inhibitory way. Primary cytoplasmic signaling sequences that act in a stimulatory manner may contain signaling motifs which are known as immunoreceptor tyrosine-based activation motifs or ITAMs. Examples of 1TAM containing primary cytoplasmic signaling sequences that are of particular use include those derived from TCR zeta, FcR gamma, FcR beta, CD3 gamma , CD3 delta, CD3 epsilon, CDS, CD22, CD79a, CD79b, and CD66d. It is particularly preferred that cytoplasmic signaling molecule in the CAR comprises a cytoplasmic signaling sequence derived from CD3 zeta.

The cytoplasmic domain of the CAR may comprise a primary signaling sequence (e.g., CD3-zeta signaling domain) by itself or combined with any other desired cytoplasmic domain(s) useful in the context of the CAR. For example, the cytoplasmic domain of the CAR may comprise a primary signaling sequence and a costimulatory signaling region. The costimulatory signaling region refers to a portion of the CAR comprising the intracellular domain of a costimulatory molecule. A costimulatory molecule is a cell surface molecule other than an antigen receptor or their ligands that is required for an efficient response of lymphocytes to an antigen. Examples of such molecules include CD27, CD28, 4- I BB (CD 137), OX40, CD30, CD40, PD- 1 , ICOS, lymphocyte function-associated antigen- 1 (LFA-1 ), CD2, CD7, LIGHT, NKG2C, B7-H3, and a ligand that specifically binds with CD83, and the like.

The cytoplasmic signaling sequences within the cytoplasmic signaling portion of the CAR may be linked to each other in a random or specified order. Optionally, a short oligopeptide linker (e.g., between 2 and 25 amino acids in length) forms the linkage. A glycine-serine doublet provides a particularly suitable linker.

Provided herein are lymphocytes engineered to express one or more chimeric antigen receptors (CARs) and/or engager molecules. Engineered cells may be generated by any suitable method in the art. The engineered lymphocytes may be generated by viral transduction of lymphocytes, (e.g., T-cell, NK cell, NKT cell, B cell, dendritic cell, etc.). Lymphocytes may beengineered to express/display one or more CARs (e.g., targeting CD123, CLL-1, and/or other antigens). Lymphocytes may be engineered to express/secrete one or more engager molecules (e.g., targeting CD123, CLL-1, and/or other antigens). Lymphocytes may be engineered to express/display one or more CARs (e.g., targeting CD123, CLL-1, and/or other antigens) and to express/secrete one or more engager molecules (e.g., targeting CD123, CLL-1, and/or other antigens).

Provided herein are nucleic acids and nucleic acid sequences encoding bispecific engager molecules and CARs as described above and cells harboring such nucleic acids. Nucleic acid molecules may be recombinant nucleic acid molecules. Nucleic acid molecules may be synthetic. Nucleic acids encoding bispecific engager molecules and CARs may comprise DNA, RNA, PNA (peptide nucleic acid), and hybrids thereof.

A nucleic acid encoding a bispecific engager molecule and/or CAR may comprise one or more regulatory sequences. For example, promoters, transcriptional enhancers and/or sequences that allow for induced expression of the polynucleotide of the disclosure may be employed. Nucleic acid molecules may be transcribed by an appropriate vector comprising a chimeric gene that allows for the transcription of the nucleic acid molecule in the cell.

A nucleic acid molecule may be a recombinantly-produced chimeric nucleic acid molecule comprising any of the aforementioned nucleic acid molecules either alone or in combination. The nucleic acid molecule may be part of a vector.

Provided herein are vectors comprising the nucleic acid molecule described herein (e.g., encoding CARs and/or engager molecules). Many suitable vectors are known to those skilled in molecular biology, the choice of which would depend on the function desired and include plasmids, cosmids, viruses, bacteriophages and other vectors used conventionally in genetic engineering. Methods that are well known to those skilled in the art can be used to construct various plasmids and vectors; see, for example, the techniques described in Sambrook et al. (1989) and Ausubel, Current Protocols in Molecular Biology, Green Publishing Associates and Wiley Interscience, N.Y. (1989), (1994). Alternatively, the polynucleotides and vectors of the disclosure are reconstituted into liposomes for delivery to target cells. A cloning vector may be used to isolate individual sequences of DNA. Relevant sequences can be transferred into expression vectors where expression of a particular polypeptide is required. Typical cloning vectors include pBluescript SK, pGEM, pUC9, pBR322 and pGBT9. Typical expression vectors include pTRE, pCAL-n-EK, pESP-1, pOP13CAT.

A vector may comprise a nucleic acid sequence that is a regulatory sequence operably linked to the nucleic acid sequence encoding a CAR and/or engager molecule described herein. Such regulatory sequences (control elements) are known to the artisan and may include a promoter, a splice cassette, translation initiation codon, and insertion site for introducing an insert into the vector. The nucleic acid molecule may be operatively linked to said expression control sequences allowing expression in eukaryotic or prokaryotic cells.

The vector may be a viral vector, such as a lentiviral vector or adenovirus associate vector.

Nucleic acids and/or vectors may be used in a cell to express encoded polypeptides (e.g., CARS, engager molecules, etc.) in the cells. The nucleic acid molecules or vectors containing the DNA sequence(s) encoding any of the CAR and/or engager constructs herein are introduced into the cells that in turn produce the polypeptide(s). The recited nucleic acid molecules and vectors may be designed for direct introduction or for introduction via liposomes, or viral vectors (e.g., adenoviral, retroviral) into a cell. The cells may be T-cells, CAR T-cells, NK cells, NKT-cells, MSCs, etc.

In accordance with the above, provided herein are methods to derive vectors, particularly plasmids, cosmids, viruses and bacteriophages used conventionally in genetic engineering that comprise a nucleic acid molecule encoding a polypeptide sequence (e.g., a CAR and/or engager) described herein. A vector may be an expression vector and/or a gene transfer or targeting vector. Expression vectors derived from viruses such as retroviruses, vaccinia virus, adeno-associated virus, herpes viruses, or bovine papilloma virus, may be used for delivery of polynucleotides and/or vectors into targeted cell populations. Methods which are well known to those skilled in the art can be used to construct recombinant vectors. Vectors are transferred into the host cells by well-known methods, which vary depending on the type of cellular host.

Provided herein are cells comprising a host cell transformed or transfected with a vector defined herein above (e.g., encoding a engager or CAR described herein). The host cell may be produced by introducing at least one of the above described vectors or at least one of the above described nucleic acid molecules into the host cell. The presence of the at least one vector or at least one nucleic acid molecule in the host may mediate the expression of a gene encoding the above described CAR and/or engager. The nucleic acid molecule or vector that is introduced in the host cell may either integrate into the genome of the host or it may be maintained extrachromosomally.

Provided herein are methods comprising culturing a host cell defined herein above under conditions allowing the introduction of the nucleic acid and/or vector. Provided herein are methods comprising culturing a host cell defined herein above under conditions allowing expression of a construct (e.g., comprising a CAR and/or engager). The cultured cells (e.g., expressing/displaying a CAR and/or expressing/secreting an engager molecule) may be provided to a subject (e.g., from which the original cells were obtained, a second subject, etc.). Conditions for the culturing of cells harboring an expression construct are known in the art.

Lymphocytes for engineering as disclosed herein may be from any suitable source. For example, a source of lymphocytes is a subject (e.g., the subject to be treated, a healthy subject, etc.). Lymphocytes can be obtained from a number of sources, including peripheral blood mononuclear cells, bone marrow, lymph node tissue, cord blood, thymus tissue, tissue from a site of infection, ascites, pleural effusion, spleen tissue, and tumors. A specific type of lymphocyte (e.g., T cell, NK cell, B cell, etc.) desired for the use described herein may be obtained by appropriate methods. Lymphocytes expressing a particular marker may be obtained by known methods (e.g., cell sorting). Cells may be cultured following isolation. Cells may be engineered using methods described herein.

Engager molecules, CARs, nucleic acid sequences, vectors, host cells, etc. as contemplated herein and/or pharmaceutical compositions comprising the same may be used for the prevention, treatment or amelioration of a cancerous disease, such as, for example AML.

Compositions herein (e.g., CAR-cells, ENG-cells, nucleic acid molecules and vectors, etc.) may be administered either alone or in any combination using standard delivery systems and methods, and in at least some aspects, together with a pharmaceutically acceptable carrier or excipient. In the case of nucleic acid molecules or vectors, they may be stably integrated into the genome of the subject.

Methods and compositions may be provided relating to the prevention, treatment or amelioration of a cancer comprising the step of administering to a subject in the need thereof an effective amount of cells harboring an engager molecule, CAR, a nucleic acid sequence, a vector, as contemplated herein and/or produced by a process as contemplated herein. When cells are administered, the engineered cells are either administered to a site of treatment or may localize at a site of treatment (e.g., cell type, tissue type, etc.).

Indications for administration of the composition(s) herein may be cancerous diseases. Examples of hematological (or hematogenous) cancers that are treated/prevented in methods herein include leukemias, including acute leukemias (such as acute lymphocytic leukemia, acute myelocytic leukemia, acute myelogenous leukemia and myeloblasts, promyeiocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myeiodysplastic syndrome, hairy cell leukemia and myelodysplasia. Examples of solid tumors that are treated/prevented in methods herein include, such as sarcomas and carcinomas, include fibrosarcoma, myxosarcoma, liposarcoma, chondrosarcoma, osteosarcoma, and other sarcomas, synovioma, mesothelioma, Ewing's tumor, leiomyosarcoma, rhabdomyosarcoma, colon carcinoma, lymphoid malignancy, pancreatic cancer, breast cancer, lung cancers, ovarian cancer, prostate cancer, hepatocellular carcinoma, squamous eel! carcinoma, basal cell carcinoma, adenocarcinoma, sweat gland carcinoma, medullary thyroid carcinoma, papillary thyroid carcinoma, pheochromocytomas sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, seminoma, bladder carcinoma, melanoma, and CNS tumors (such as a glioma (such as brainstem glioma and mixed gliomas), glioblastoma (also known as glioblastoma multiforme) astrocytoma, CNS lymphoma, germinoma, medu!loblastoma, Schwannoma craniopharyogioma, ependymoma, pineaioma, hemangioblastoma, acoustic neuroma, oligodendroglioma, menangioma, neuroblastoma, retinoblastoma and brain metastases).

The disclosure further encompasses co-administration protocols with other compounds, e.g. bispecific antibody constructs, targeted toxins or other blocking or functional antibodies or compounds, which act via immune cells. The clinical regimen for co-administration may encompass co-administration at the same time, before or after the administration of the other component. Particular combination therapies include chemotherapy, radiation, surgery, hormone therapy, or other types of immunotherapy. Many chemotherapeutics are presently known in the art and can be used in combination with the compounds described herein. The chemotherapeutic may be selected from the group consisting of mitotic inhibitors, alkylating agents, anti-metabolites, intercalating antibiotics, growth factor inhibitors, cell cycle inhibitors, enzymes, topoisomerase inhibitors, biological response modifiers, anti-hormones, angiogenesis inhibitors, and anti-androgens.

The engineered lymphocytes described herein may be co-administered with one or more chemotherapeutics. Chemotherapies for use with the engineered lymphocytes described herein include all classes of chemotherapeutic agents, such as, alkylating agents, antimetabalites, plant alkaloids, antibiotics, hormonal agents, and miscellaneous anticancer drugs. Specific agents include, for example, abraxane, altretamine, docetaxel, herceptin, methotrexate, novantrone, zoladex, cisplatin (CDDP), carboplatin, procarbazine, mechlorethamine, cyclophosphamide, camptothecin, ifosfamide, melphalan, chlorambucil, busulfan, nitrosurea, dactinomycin, daunorubicin, doxorubicin, bleomycin, plicomycin, mitomycin, etoposide (VP16), tamoxifen, raloxifene, estrogen receptor binding agents, taxol, gemcitabine, fuldarabine, navelbine, farnesyl-protein tansferase inhibitors, transplatinum, 5-fluorouracil, vincristin, and vinblastin, or any analog or derivative variant of the foregoing and also combinations thereof. Chemotherapy may be employed before, during and/or after administration of engineered lymphocytes.

The engineered lymphocytes described herein may be co-administered with radiotherapy, methods of which are understood in the field. Radiotherapy may be employed before, during and/or after administration of engineered lymphocytes.

The engineered lymphocytes described herein may be co-administered with non-immune based targeted therapies, such as, agents that inhibit signaling pathways such WNT, p53, and/or RB-signaling pathways. Other examples include agents that inhibit tyrosine kinases, BRAF, STAT3, c-met, regulate gene expression, induce cell death or block blood vessel formation. Examples of specific agents include imatinib mesylate, dasatinib, nilotinib, bosutinib, lapatinib, gefinitib, erlotinib, tensirolimus, everolimus, vemurafenib, crizotinib, vorinostat, romidepsin, bexarotene, alitrionin, tretionin, bortezomib, carfilzomib, pralatrexate, sorafenib, sunitinib, pazopanib, regorafenib, or cabozantinib. Non-immune based targeted therapy may be employed before, during and/or after administration of engineered lymphocytes.

The engineered lymphocytes described herein may be co-administered with an immunotherapy. Immunotherapeutics generally rely on the use of immune effector cells and molecules to target and destroy cancer cells. The immune effector may be, for example, an antibody specific for some marker on the surface of a tumor cell. The antibody alone may serve as an effector of therapy or it may recruit other cells to actually effect-cell killing. The antibody may also prevent cancer immunoevasion or immunosuppression. The antibody also may be conjugated to a drug or toxin (chemotherapeutic, radionuclide, ricin A chain, cholera toxin, pertussis toxin, etc.) and serve merely as a targeting agent. Alternatively, the effector may be a lymphocyte carrying a surface molecule that interacts, either directly or indirectly, with a tumor cell target. Various effector cells include cytotoxic T-cells, NKT cells, and NK cells. Immunotherapy may be employed before, during and/or after administration of engineered lymphocytes. Engineered lymphocytes may be co-administered with an immune checkpoint inhibitor (e.g., anti-PD1, anti-PDL1, anti-CTLA-4, etc.).

The engineered lymphocytes described herein may be co-administered with a gene therapy in which a therapeutic polynucleotide is administered before, after, or at the same time as the engineered lymphocytes described herein. A variety of expression products are encompassed, including inducers of cellular proliferation, inhibitors of cellular proliferation, or regulators of programmed cell death.

The engineered lymphocytes described herein may be administered before, during, and/or after surgery. Surgeries include resection in which all or part of cancerous tissue is physically removed, excised, and/or destroyed. Tumor resection refers to physical removal of at least part of a tumor. In addition to tumor resection, treatment by surgery includes laser surgery, cryosurgery, electrosurgery, and miscopically controlled surgery (Mohs' surgery). It is further contemplated that methods herein may be used in conjunction with removal of superficial cancers, precancers, or incidental amounts of normal tissue.

The engineered lymphocytes described herein may be co-administered with other agents to improve the therapeutic efficacy of treatment.

Engineered lymphocytes described herein may be provided as part of a kit or system along with one or more additional components, such as instructions, devices for administration, additional therapeutic agents, diagnostic agents, research agents, etc.

### EXPERIMENTAL

### Example 1

### CLL1-ENG

Experiments conducted demonstrate that CLL-1 is expressed on a range of human myeloid leukemia cell lines and primary acute myeloid leukemia samples. Flow cytometry analysis of established KG1a, MOLM-13, MV-4-11, and OCIAML-3, human myeloid leukemia cell lines using anti-human CLL-1-FITC (Figure 1A) and analysis of primary human acute myeloid leukemia samples (Figure 1B) demonstrate the presence of CLL-1 on the surface of human myeloid leukemia cells.

Experiments conducted demonstrate that human T cells can be transduced to express bispecific engager molecules with an antigen-recongition domain specific for CLL-1 and an activation domain specific for CD3ε, an integral component of the T-cell receptor complex (CLL1-ENG; Figure 2A). Flow cytometry analysis demonstrates the successful transduction of human T cells with the CLL1-ENG retroviral vector..

Experiments conducted to demonstrate of CLL1-ENG T cell activation and functional activity versus CLL-1-positive target cells (Figure 3). Co-culture of CLL1-ENG T cells and CLL1+ targets cells results in T cell activation. Measurement of IFNγ and IL-2, cytokines secreted by activated T cells, in the cell culture supernatant of selectively activated CLL1-ENG T cells proves specific T-cell activation (Fig. 3A, B). Co-culture of CLL1-ENG T cells and the CLL-1+ target cells MV-4-11 and OCI-AML-3 results in death of target cells. Co-culture of CLL-1 ENG T cells and the CLL-1 negative MOLM-13 AML cell line does not result in MOLM-13 cell death. This is demonstrated by measurement of cell viability following CLL1-ENG T cell and AML cell co-culture with bioluminescence measurement in AML lines stably transduced to express firefly Luciferase and treated with D-Luciferin (Fig. 3C), as well as FACS analysis following co-culture for the AML surface antigen CD33 and the T cell antigen CD3 (Fig 3D,E).

Experiments conducted to demonstrate CLL1-ENG activation of autologous primary T cells versus acute myeloid leukemia (Figure 4). Primary peripheral blood samples containing both AML cells and T cells were plated below a transwell. The transwell allows for the transmission of small molecules, such as the CLL1-ENG, but not cells. When CLL-1 ENG T cells that secrete CLL1 ENG are plated in the top transwell, the ENG molecule travels through the membrane to activate primary autologous T cells to kill AML blasts present in the culture. Media alone or non-transduced T cells plated in the upper chamber do not activate autologous T cells to kill AML blasts.

### Example 2

### CD123-CAR

Experiments conducted CD123-CAR expression. FACS analysis performed following retroviral transduction of activated T-cells with retroviral construct containing CD123 scFv, CD28 co-stimulatory region, and CD3ζ intracellular signaling domain. As the retroviral vector construct contains the coding sequence for the fluorescent protein mOrange downstream of an IRES, FACS analysis for mOrange expression proves high efficiency transduction of T cells.

Experiments conducted demonstrating CD123-CAR T cell activation and cytotoxicity versus CD123+ target cells. Co-culture of CD123-CAR T cells and CD123+ targets cells (MOLM-13, primary AML) results in T cell activation. Measurement of IFNy and IL-2, cytokines secreted by activated T cells, in the cell culture supernatant of selectively activated CD123-CAR T cells proves specific T-cell activation. CD123-CAR T cells in culture with CD123-negative targets (no target, K562) does not result in cytokine secretion. CD123-CAR T cells, when cultured with CD123_, CD33+ MOLM-13 AML cells efficiently kill target cells as measured by FACS analysis for CD33+ cells. Non-transduced T cells exhibit no cytotoxicity vs. target cells.

### Example 3

### CD123(CAR)/CLL-1(ENG) T cells

### Generation and functional characterization of CD123 and CLL-1 targeted T cells

Expression of CD123 and CLL-1 were measured on AML cell lines and primary myeloid blasts with flow cytometric analysis (Fig. 7A, B, C). A codon-optimized CD123- or CLL-1-ENG containing of a CD123- or CLL1-specific scFv (Du et al. J Immunother 30, 607-613 (2007).; Abo & Korver, US 8536310 (2013).), a short serine-glycine linker, and an scFv specific for CD3ε was subcloned into a retroviral vector. Transduction of normal donors was both efficient and robust.

The proven effective CD123-specific scFv are used to generate CD123-CAR T cells and Experiments are conducted using CD123ζ-CAR T against CD123+ and CD123- target cells. CD123ζ-CAR is then co-expressed with CLL-1-ENG to determine the ability of these cells to (i) produce cytokines, (ii) proliferate, (iii) kill AML tumor cells *in vitro,* and (iv) persist with ongoing anti-tumor activity in our established xenograft models *in vivo.* Standard *in vitro* immunological assays and MOLM-13.ffLuc NSG (NOD-scid IL2Rg^{null}) leukemia model for *in vivo* studies are used for evaluations, as well as primary human leukemia xenografts established with patient samples (HUM000105312). An OCI-AML-3.ffLuc cell line has been prepared to facilitate comparative recognition of CLL-1+ (OCI-AML-3) and CLL-1- (MOLM-13) leukemias. Experiments will also evaluate downstream TCR signaling to differentiate this from signaling through the chimeric receptor. Experiments include intracellular cytokine staining (mOrange+, transduced; mOrange-, bystander T cell) as well as Western blot analysis. Physically separation of ENG-T from CAR-T using a transwell format is achievable, and cells can be sorted prior to analysis. The effect of ENG and CAR activation is separated to compare and contrast the contribution of each to AML pathogenic control.

### Example 4

### Demonstration of CLL-ENG activity in vivo

Experiments were conducted to demonstrate CLL-ENG activity in vivo (FIG. 8). Mice were administered leukemia cells and then were given either no treatment, treated with control ENG-T cells, or treated with CLL-ENG T cells. Leukemia progression was monitored. The results demonstrate that the administration of the CLL-ENG T cells prolonged survival compared to both of the controls.

### Example 5

### Co-expression of CLL-ENG and CD123-CAR in T cells

Experiments were conducted to demonstrate co-expression of CLL-ENG and CD123-CAR in T cells. T cells were transduced with retroviral vectors containing either CLL-ENG paired with the flurophore mOrange, or a CD123 chimeric receptor paired with the fluorophore ZsGreen. Successful genetic modification was evaluated with FACS analysis and detection of mOrange and/or ZsGreen. Dual modified cells were detected (FIG. 9D; circle).

### Example 6

### Enhanced activation with dual T cell modification

Experiments were conducted to demonstrate enhancement of activation by dual T cell modification. Target cells (CD123-negative/CLL1-negative or CD123-positive/CLL1-positive) were plated in co-culture with unmodified, control-ENG, CLL-ENG, CD123-CAR, or CLL-ENG/CD123-CAR T cells. After 24 hours of culture, supernatant was harvested and assessed for the presence of Interferon-gamma as a marker of T cell activation. Significant enhancement of T cell activation is observed by the dual-modified cells (FIG. 10).

### Example 7

### Lack of toxicity to stem and progenitor hematopoietic cells by CLL1-targeting

Experiments were conducted to assess toxicity to stem and progenitor hematopoietic cells by CLL1-targeting (FIG. 11). Bone marrow mononuclear cells (BMMC), isolated from healthy donor bone marrow via a density gradient, were plated and incubated with T cells (unmodified (NT), CD19-ENG, CLL-ENG, and CD123-ENG). After incubation, cells were plated and incubated in semisolid media containing growth factors. The resulting colonies were counted manually, identified as CFU-E or CFU-GM, and the count was then normalized to that of identical conditions without T cells present.

### Example 8

### CAR-NK modification

Experiments were conducted to demonstrate the CAR modification of NK cells (FIG 12). Healthy donor NK cells were selected from peripheral blood mononuclear cells and transduced with retroviral vectors encoding chimeric receptors. Receptors contain an ectodomain to bind CD123 and an endodomain consisting of a combination of: TCRζ, DAP10, DAP12, 41BB, and/or CD28. CAR expression measured on D5 and D18 post-transduction with FACS analysis.

### Example 9

### Demonstration of specific NK- cell activation

Experiments were conducted to demonstrate activation by CAR-NK cells.

Target cells were plated in co-culture with unmodified, 41BB.ζ, DAP10.41BB, or DAP12.41BB CAR-NK cells. After 24 hours of culture, supernatant was harvested and assessed for the presence of Interferon-gamma as a marker of NK cell activation. Significant NK cell activation was measured in MV-4-11 and Molm-13 target cells (FIG. 13A).

NK cells and target (stably expressing firefly luciferase) were co-cultured for 18 hours, and bioluminescent imaging (BLI) was measured as a correlate to living cells in culture. Cytoxicity was then calculated as a comparison between cells identically treated without NK cells present (FIG. 13B). No difference was seen in cytoxicity of unmodified NK or NK-CAR for Raji (CD123-negative). Significantly increased killing (p<0.0001) was evident when comparing unmodified to CAR-NK for Molm-13 (CD123-positive).

### Example 10

### In vivo activation of NK-CAR

Experiments were conducted to demonstrate *in vivo* activation of NK-CAR. Mice were administered leukemia cells on day 0 followed by no treatment or 5e6 CD123-CAR NK cells on day 7. Leukemia progression was monitored with detection of whole bioluminescence per mouse. Representative images of mice on day 28 post-leukemia injection are shown (FIG 14). Line graph is representative of bioluminescence measured per mouse on indicated day post-leukemia injection (FIG. 14).

### Example 11

### Dual-NK modification

Experiments were conducted to demonstrate dual-NK modification (FIG. 15). NK cells were transduced with retroviral vectors containing either CLL-ENG paired with the fluorophore mOrange, or a CD123 chimeric receptor. Successful genetic modification was evaluated with FACS analysis and detection of mOrange and/or CAR expression.

### REFERENCES

1. Lowenberg, B., Downing, J. R. & Burnett, A. Acute myeloid leukemia. N Engl J Med 341, 1051-1062, doi:10.1056/NEJM199909303411407 (1999).
2. Woods, W. G. Curing childhood acute myeloid leukemia (AML) at the half-way point: promises to keep and miles to go before we sleep. Pediatr Blood Cancer 46, 565-569, doi:10.1002/pbc.20646 (2006).
3. Gorman, M. F. et al. Outcome for children treated for relapsed or refractory acute myelogenous leukemia (rAML): a Therapeutic Advances in Childhood Leukemia (TACL) Consortium study. Pediatr Blood Cancer 55, 421-429, doi:10.1002/pbc.22612 (2010).
4. Kalos, M. et al. T cells with chimeric antigen receptors have potent antitumor effects and can establish memory in patients with advanced leukemia. Sci Transl Med 3, 95ra73, doi:10.1126/scitranslmed.3002842 (2011).
5. Kochenderfer, J. N. et al. B-cell depletion and remissions of malignancy along with cytokineassociated toxicity in a clinical trial of anti-CD19 chimeric-antigen-receptor-transduced T cells. Blood 119, 2709-2720, doi:10.1182/blood-2011-10-384388 (2012).
6. Kochenderfer, J. N. et al. Donor-derived CD19-targeted T cells cause regression of malignancy persisting after allogeneic hematopoietic stem cell transplantation. Blood 122, 4129-4139, doi:10.1182/blood-2013-08-519413 (2013).
7. Brentjens, R. J. et al. CD19-targeted T cells rapidly induce molecular remissions in adults with chemotherapy-refractory acute lymphoblastic leukemia. Sci Transl Med 5, 177ra138, doi:10.1126/scitranslmed.3005930 (2013).
8. Maude, S. L. et al. Chimeric antigen receptor T cells for sustained remissions in leukemia. N Engl J Med 371, 1507-1517, doi:10.1056/NEJMoa1407222 (2014).
9. Kochenderfer, J. N. et al. Chemotherapy-refractory diffuse large B-cell lymphoma and indolent B-cell malignancies can be effectively treated with autologous T cells expressing an anti-CD19 chimeric antigen receptor. J Clin Oncol 33, 540-549, doi:10.1200/JCO.2014.56.2025 (2015).
10. Lee, D. W. et al. T cells expressing CD19 chimeric antigen receptors for acute lymphoblastic leukaemia in children and young adults: a phase 1 dose-escalation trial. Lancet 385, 517-528, doi:10.1016/S0140-6736(14)61403-3 (2015).
11. Grupp SA et al. T cells engineered with a chimeric antigen receptor (CAR) targeting CD19 (CTL019) have long term persistence and induce durable remissions in children with relapsed, refractory ALL. Blood 124 (2014).
12. Park JH et al. Efficacy and safety of CD19-targeted 19-28z CAR modified T cells in adult patients with relapsed or refractory B-ALL. J Clin Oncol 33 (2015).
13. Sotillo, E. et al. Convergence of Acquired Mutations and Alternative Splicing of CD19 Enables Resistance to CART-19 Immunotherapy. Cancer Discov 5, 1282-1295, doi:10.1158/2159- 8290.CD-15-1020 (2015).
14. Munoz, L. et al. Interleukin-3 receptor alpha chain (CD123) is widely expressed in hematologic malignancies. Haematologica 86, 1261-1269 (2001).
15. Ehninger, A. et al. Distribution and levels of cell surface expression of CD33 and CD123 in acute myeloid leukemia. Blood Cancer J 4, e218, doi:10.1038/bcj.2014.39 (2014).
16. Testa, U., Pelosi, E. & Frankel, A. CD 123 is a membrane biomarker and a therapeutic target in hematologic malignancies. Biomark Res 2, 4, doi:10.1186/2050-7771-2-4 (2014).
17. Mardiros, A. et al. T cells expressing CD123-specific chimeric antigen receptors exhibit specific cytolytic effector functions and antitumor effects against human acute myeloid leukemia. Blood 122, 3138-3148, doi:10.1182/blood-2012-12-474056 (2013).
18. Tettamanti, S. et al. Targeting of acute myeloid leukaemia by cytokine-induced killer cells redirected with a novel CD123-specific chimeric antigen receptor. Br J Haematol 161, 389-401, doi:10.1111/bjh.12282 (2013).
19. Pizzitola, I. et al. Chimeric antigen receptors against CD33/CD123 antigens efficiently target primary acute myeloid leukemia cells in vivo. Leukemia 28, 1596-1605, doi:10.1038/leu.2014.62 (2014).
20. Gill, S. et al. Preclinical targeting of human acute myeloid leukemia and myeloablation using chimeric antigen receptor-modified T cells. Blood 123, 2343-2354, doi:10.1182/blood-2013-09-529537 (2014).
21. Kenderian, S. S. et al. CD33-specific chimeric antigen receptor T cells exhibit potent preclinical activity against human acute myeloid leukemia. Leukemia 29, 1637-1647, doi:10.1038/leu.2015.52 (2015).
22. van Rhenen, A. et al. The novel AML stem cell associated antigen CLL-1 aids in discrimination between normal and leukemic stem cells. Blood 110, 2659-2666, doi:10.1182/blood-2007-03- 083048 (2007).
23. Larsen, H. O., Roug, A. S., Just, T., Brown, G. D. & Hokland, P. Expression of the hMICL in acute myeloid leukemia-a highly reliable disease marker at diagnosis and during follow-up. Cytometry B Clin Cytom 82, 3-8, doi:10.1002/cyto.b.20614 (2012).
24. Roug, A. S. et al. hMICL and CD123 in combination with a CD45/CD34/CD117 backbone - a universal marker combination for the detection of minimal residual disease in acute myeloid leukaemia. Br J Haematol 164, 212-222, doi:10.1111/bjh.12614 (2014).
25. Hegde, M. et al. Combinational targeting offsets antigen escape and enhances effector functions of adoptively transferred T cells in glioblastoma. Mol Ther 21, 2087-2101, doi:10.1038/mt.2013.185 (2013).
26. Kloss, C. C., Condomines, M., Cartellieri, M., Bachmann, M. & Sadelain, M. Combinatorial antigen recognition with balanced signaling promotes selective tumor eradication by engineered T cells. Nat Biotechnol 31, 71-75, doi:10.1038/nbt.2459 (2013).
27. Wu, C. Y., Roybal, K. T., Puchner, E. M., Onuffer, J. & Lim, W. A. Remote control of therapeutic T cells through a small molecule-gated chimeric receptor. Science 350, aab4077, doi:10.1126/science.aab4077 (2015).
28. Roybal, K. T. et al. Precision Tumor Recognition by T Cells With Combinatorial Antigen-Sensing Circuits. Cell 164, 770-779, doi:10.1016/j.cell.2016.01.011 (2016).
29. Stephan, M. T. et al. T cell-encoded CD80 and 4-1BBL induce auto- and transcostimulation, resulting in potent tumor rejection. Nat Med 13, 1440-1449, doi:10.1038/nm1676 (2007).
30. Hoyos, V. et al. Engineering CD19-specific T lymphocytes with interleukin-15 and a suicide gene to enhance their anti-lymphoma/leukemia effects and safety. Leukemia 24, 1160-1170, doi:10.1038/leu.2010.75 (2010).
31. Pegram, H. J. et al. Tumor-targeted T cells modified to secrete IL-12 eradicate systemic tumors without need for prior conditioning. Blood 119, 4133-4141, doi:10.1182/blood-2011-12- 400044 (2012).
32. Curran, K. J. et al. Enhancing antitumor efficacy of chimeric antigen receptor T cells through constitutive CD40L expression. Mol Ther 23, 769-778, doi:10.1038/mt.2015.4 (2015).
33. Mack, M., Riethmuller, G. & Kufer, P. A small bispecific antibody construct expressed as a functional single-chain molecule with high tumor cell cytotoxicity. Proc Natl Acad Sci USA 92, 7021-7025 (1995).
34. Topp, M. S. et al. Safety and activity of blinatumomab for adult patients with relapsed or refractory B-precursor acute lymphoblastic leukaemia: a multicentre, single-arm, phase 2 study. Lancet Oncol 16, 57-66, doi:10.1016/S1470-2045(14)71170-2 (2015).
35. Topp, M. S. et al. Long-term follow-up of hematologic relapse-free survival in a phase 2 study of blinatumomab in patients with MRD in B-lineage ALL. Blood 120, 5185-5187, doi:10.1182/blood-2012-07-441030 (2012).
36. Topp, M. S. et al. Phase II trial of the anti-CD19 bispecific T cell-engager blinatumomab shows hematologic and molecular remissions in patients with relapsed or refractory B-precursor acute lymphoblastic leukemia. J Clin Oncol 32, 4134-4140, doi:10.1200/JCO.2014.56.3247 (2014).
37. Teachey, D. T. et al. Cytokine release syndrome after blinatumomab treatment related to abnormal macrophage activation and ameliorated with cytokine-directed therapy. Blood 121, 5154-5157, doi:10.1182/blood-2013-02-485623 (2013).
38. Iwahori, K. et al. Engager T cells: a new class of antigen-specific T cells that redirect bystander T cells. Mol Ther 23, 171-178, doi:10.1038/mt.2014.156 (2015).
39. Bonifant, C. L. et al. CD123-Engager T cells as a Novel Immunotherapeutic for Acute Myeloid Leukemia. submitted*.*
40. Zal, T. & Chodaczek, G. Intravital imaging of anti-tumor immune response and the tumor microenvironment. Semin Immunopathol 32, 305-317, doi:10.1007/s00281-010-0217-9 (2010).
41. Savoldo, B. et al. CD28 costimulation improves expansion and persistence of chimeric antigen receptor-modified T cells in lymphoma patients. J Clin Invest 121, 1822-1826, doi:10.1172/JCI46110 (2011).
42. Du, X., Ho, M. & Pastan, I. New immunotoxins targeting CD123, a stem cell antigen on acute myeloid leukemia cells. J Immunother 30, 607-613, doi:10.1097/CJI.0b013e318053ed8e (2007).
43. Abo, A. & Korver, W. Antibodies to CLL-1. USA patent US 8536310 B2 (2013).
44. Mandl, J. N. et al. Quantification of lymph node transit times reveals differences in antigen surveillance strategies of naive CD4+ and CD8+ T cells. Proc Natl Acad Sci U S A 109, 18036- 18041, doi:10.1073/pnas.1211717109 (2012).
45. Huang, A. Y. et al. Viewing transplantation immunology through today's lens: new models, new imaging, and new insights. Biol Blood Marrow Transplant 19, S44-51, doi:10.1016/j.bbmt.2012.10.020 (2013).
46. Myers, J. T., Barkauskas, D. S. & Huang, A. Y. Dynamic Imaging of Marrow-Resident Granulocytes Interacting with Human Mesenchymal Stem Cells upon Systemic Lipopolysaccharide Challenge. Stem Cells Int 2013, 656839, doi:10.1155/2013/656839 (2013).
47. Wang, W. et al. Aberrant Notch signaling in the bone marrow microenvironment of acute lymphoid leukemia suppresses osteoblast-mediated support of hematopoietic niche function. Cancer Res, doi:10.1158/0008-5472.CAN-15-2092 (2016).

## Claims

1. Engineered lymphocytes for use in a method of treating acute myeloid leukemia (AML) in a subject, wherein said engineered lymphocytes express a polypeptide construct comprising an antigen-recognition domain and an activation domain, wherein the antigen-recognition domain binds C-type lectin-like molecule-1 (CLL-1) displayed on a malignant myeloblast cell, and wherein the activation domain triggers an immune response against the malignant myeloblast cell by the engineered lymphocytes and/or native T cells upon binding of the antigen-recognition domain to CLL-1, and wherein the polypeptide construct is a bispecific engager molecule comprising a single chain variable fragment (scFv) activation domain that binds CD3 and an scFv antigen-recognition domain that binds CLL-1.

2. The engineered lymphocytes for use of claim 1, wherein the bispecific engager molecule is secreted from the engineered lymphocytes.

3. The engineered lymphocytes for use of claim 1, further defined by one of the following features:
i) the engineered lymphocytes are engineered T-cells, engineered NK cells, or engineered NKT cells;
ii) wherein the method comprises obtaining lymphocytes from the subject, genetically engineering the subject's lymphocytes to express the polypeptide construct, and culturing the resulting engineered lymphocytes.

4. An engineered lymphocyte comprising a polynucleotide that encodes a bispecific engager molecule, said bispecific engager molecule comprising a scFv activation domain that binds CD3 and a scFv antigen-recognition domain that binds CLL-1, wherein preferably the activation domain and antigen-recognition domain are single chain variable fragments tethered to each other by a linker domain.

5. The engineered lymphocyte of claim 4, further defined by one of the following features:
i) the engineered lymphocyte expresses the bispecific engager molecule from the polynucleotide and the bispecific engager molecule is secreted from the engineered lymphocyte;
ii) the lymphocyte is a T cell;
iii) the lymphocyte is an NK cell.

6. Engineered lymphocyte of one of claims 4-5 for use in the treatment of a subject.

7. Engineered lymphocyte for use of claim 6, wherein the subject suffers from cancer, wherein the subject preferably suffers from leukemia, wherein the subject further preferably suffers from acute myeloid leukemia (AML).

8. Engineered lymphocyte for use of claim 6, further defined by one of the following features:
i) the engineered lymphocyte expresses and secretes the bispecific engager molecule within the subject;
ii) the bispecific engager molecule binds to malignant cells displaying CLL-1 and T cells, thereby activating the T cells to attack the malignant cells displaying CLL-1.

## Patentansprüche

1. Manipulierte Lymphozyten zur Verwendung in einem Verfahren zur Behandlung von akuter myeloischer Leukämie (AML) bei einem Subjekt, wobei die manipulierten Lymphozyten ein Polypeptidkonstrukt exprimieren, das eine Antigen-Erkennungsdomäne und eine Aktivierungsdomäne umfasst, wobei die Antigen-Erkennungsdomäne an ein C-Typ-Lektin-ähnliches Molekül-1 (CLL-1) bindet, das auf einer malignen Myeloblastenzelle exprimiert wird, und wobei die Aktivierungsdomäne eine Immunantwort gegen die maligne Myeloblastenzelle durch die manipulierten Lymphozyten und/oder native T-Zellen nach Bindung der Antigenerkennungsdomäne an CLL-1 auslöst, und wobei das Polypeptidkonstrukt ein bispezifisches Engager-Molekül ist, das eine Aktivierungsdomäne aus einem einzelkettigen variablen Fragment (scFv) umfasst, das an CD3 bindet, und eine scFv-Antigenerkennungsdomäne, die an CLL-1 bindet.

2. Manipulierte Lymphozyten zur Verwendung nach Anspruch 1, wobei das bispezifische Engager-Molekül aus den manipulierten Lymphozyten sekretiert wird.

3. Manipulierte Lymphozyten zur Verwendung nach Anspruch 1, die des Weiteren durch eines der folgenden Merkmale definiert sind:
i) die manipulierten Lymphozyten sind manipulierte T-Zellen, manipulierte NK-Zellen oder manipulierte NKT-Zellen;
ii) wobei das Verfahren umfasst: Gewinnen von Lymphozyten aus dem Subjekt, genetisches Manipulieren der Lymphozyten des Subjekts, um das Polypeptidkonstrukt zu exprimieren, und Kultivieren der resultierenden manipulierten Lymphozyten.

4. Manipulierter Lymphozyt, das ein Polynukleotid umfasst, das ein bispezifisches Engager-Molekül kodiert, wobei das bispezifische Engager-Molekül eine scFv-Aktivierungsdomäne, die an CD3 bindet, und eine scFv-Antigenerkennungsdomäne, die an CLL-1 bindet, umfasst, wobei vorzugsweise die Aktivierungsdomäne und die Antigenerkennungsdomäne einkettige variable Fragmente sind, die durch eine Linker-Domäne miteinander verbunden sind.

5. Manipulierter Lymphozyt nach Anspruch 4, der des Weiteren durch eines der folgenden Merkmale definiert ist:
i) der manipulierte Lymphozyt exprimiert das bispezifische Engager-Molekül aus dem Polynukleotid und das bispezifische Engager-Molekül wird aus dem manipulierten Lymphozyten sekretiert;
ii) der Lymphozyt ist eine T-Zelle;
iii) der Lymphozyt ist eine NK-Zelle.

6. Manipulierte Lymphozyten nach einem der Ansprüche 4-5 zur Verwendung bei der Behandlung eines Subjekts.

7. Manipulierte Lymphozyten zur Verwendung nach Anspruch 6, wobei das Subjekt an Krebs leidet, wobei das Subjekt vorzugsweise an Leukämie leidet, wobei das Subjekt des Weiteren bevorzugt an akuter myeloischer Leukämie (AML) leidet.

8. Manipulierte Lymphozyten zur Verwendung nach Anspruch 6, die des Weiteren durch eines der folgenden Merkmale definiert sind:
i) die manipulierten Lymphozyten exprimieren und sezernieren das bispezifische Engager-Molekül innerhalb des Subjekts;
ii) das bispezifische Engager-Molekül bindet an maligne Zellen, die CLL-1 exprimieren, und an T-Zellen, wodurch die T-Zellen aktiviert werden, die malignen Zellen, die CLL-1 exprimieren, anzugreifen.

## Revendications

1. Lymphocytes modifiés pour une utilisation dans une méthode de traitement de la leucémie aiguë myéloïde (LAM) chez un sujet, lesdits lymphocytes modifiés exprimant une construction polypeptidique comprenant un domaine de reconnaissance d'antigène et un domaine d'activation, le domaine de reconnaissance d'antigène se liant à la molécule de type lectine de type C-1 (CLL-1) présentée sur un myéloblaste malin, et le domaine d'activation déclenchant une réponse immunitaire contre le myéloblaste malin par les lymphocytes modifiés et/ou les lymphocytes T natifs lors de la liaison du domaine de reconnaissance d'antigène à CLL-1, et la construction polypeptidique étant une molécule d'engagement bispécifique comprenant un domaine d'activation de fragment variable monocaténaire (scFv) qui se lie à CD3 et un domaine de reconnaissance d'antigène scFv qui se lie à CLL-1.

2. Lymphocytes génétiquement modifiés selon la revendication 1, la molécule d'engagement bispécifique étant sécrétée par les lymphocytes modifiés.

3. Lymphocytes modifiés selon la revendication 1, définis plus avant par l'une des caractéristiques suivantes :
i) les lymphocytes modifiés sont des lymphocytes T modifiés, des cellules NK modifiées ou des cellules NKT modifiées ;
ii) la méthode comprend l'obtention de lymphocytes à partir du sujet, la modification génétique des lymphocytes du sujet pour exprimer la construction polypeptidique, et la culture des lymphocytes modifiés obtenus.

4. Lymphocyte modifié comprenant un polynucléotide qui code pour une molécule d'engagement bispécifique, ladite molécule d'engagement bispécifique comprenant un domaine d'activation scFv qui se lie à CD3 et un domaine de reconnaissance d'antigène scFv qui se lie à CLL-1, de préférence, le domaine d'activation et le domaine de reconnaissance d'antigène étant des fragments variables monocaténaires reliés entre eux par un domaine de lieur.

5. Lymphocyte modifié selon la revendication 4, défini plus avant par l'une des caractéristiques suivantes :
i) le lymphocyte modifié exprime la molécule d'engagement bispécifique à partir du polynucléotide et la molécule d'engagement bispécifique est sécrétée par le lymphocyte modifié ;
ii) le lymphocyte est un lymphocyte T ;
iii) le lymphocyte est une cellule NK.

6. Lymphocyte modifié selon l'une des revendications 4 à 5, pour une utilisation dans le traitement d'un sujet.

7. Lymphocyte modifié selon la revendication 6, le sujet souffrant d'un cancer,
de préférence, le sujet souffrant d'une leucémie, plus préférentiellement, le sujet souffrant d'une leucémie aiguë myéloïde (LAM).

8. Lymphocyte modifié selon la revendication 6, définis plus avant par l'une des caractéristiques suivantes :
i) le lymphocyte modifié exprime et sécrète la molécule d'engagement bispécifique dans le sujet ;
ii) la molécule d'engagement bispécifique se lie aux cellules malignes présentant CLL-1 et aux lymphocytes T, activant ainsi les lymphocytes T pour attaquer les cellules malignes présentant CLL-1.
